# EUROPÄISCHE PATENTSCHRIFT

(11) **EP 2 935 602 B1**
(45) Veröffentlichungstag und Bekanntmachung des Hinweises auf die Patenterteilung: **21.02.2018**
(21) Anmeldenummer: 13810940.0
(22) Anmeldetag: 18.12.2013
(51) Int. Cl.: C12P 13/00, C12N 15/52, C12N 9/00, C12N 9/10, C12N 9/12

(54) **HERSTELLUNG VON AMINEN UND DIAMINEN AUS EINER CARBONSÄURE ODER DICARBONSÄURE ODER EINES MONOESTERS DAVON**
PREPARATION OF AMINES AND DIAMINES FROM A CARBOXYLIC ACID OR DICARBOXYLIC ACID OR A MONOESTER THEREOF
FABRICATION D'AMINES ET DE DIAMINES À PARTIR D'ACIDE CARBONIQUE OU D'ACIDE DE DICARBONE OU UN MONO-ESTER COMPOSÉ DE CEUX-CI

(30) Priorität: 21.12.2012 EP 12199048
(43) Veröffentlichungstag der Anmeldung: 28.10.2015
(73) Patentinhaber: Evonik Degussa GmbH, 45128 Essen (DE)
(72) Erfinder: SCHAFFER, Steffen, 45699 Herten (DE); CORTHALS, Jasmin, 44879 Bochum (DE); WESSEL, Mirja, 44799 Bochum (DE); HENNEMANN, Hans-Georg, 50181 Bedburg (DE); HÄGER, Harald, 59348 Lüdinghausen (DE); VOLLAND, Michael, 48249 Dülmen (DE); ROOS, Martin, 45721 Haltern am See (DE)
(86) Internationale Anmeldenummer: PCT/EP2013/077069
(87) Internationale Veröffentlichungsnummer: WO 2014/095986

(56) Entgegenhaltungen:
- WO-A1-2009/077461
- WO-A1-2011/131420
- WO-A1-2012/025629
- WO-A1-2013/135650
- WO-A2-2010/135624
- URSULA KAULMANN ET AL: "Substrate spectrum of [omega]-transaminase from Chromobacterium violaceum DSM30191 and its potential for biocatalysis", ENZYME AND MICROBIAL TECHNOLOGY, Bd. 41, Nr. 5, 1. Oktober 2007 (2007-10-01), Seiten 628-637, XP055026143, ISSN: 0141-0229, DOI: 10.1016/j.enzmictec.2007.05.011
- MANFRED SCHREWE ET AL: "Direct Terminal Alkylamino-Functionalization via Multistep Biocatalysis in One Recombinant Whole-Cell Catalyst", ADVANCED SYNTHESIS & CATALYSIS, Bd. 355, Nr. 9, 22. Februar 2013 (2013-02-22), Seiten 1693-1697, XP55116393, ISSN: 1615-4150, DOI: 10.1002/adsc.201200958
- ROBERT C. SIMON ET AL: "Recent Developments of Cascade Reactions Involving [omega]-Transaminases", ACS CATALYSIS, Bd. 4, Nr. 1, 10. Dezember 2013 (2013-12-10), Seiten 129-143, XP055116474, ISSN: 2155-5435, DOI: 10.1021/cs400930v

## Beschreibung

Die Erfindung betrifft einen Ganzzellkatalysator, der eine rekombinante α-Dioxygenase und/oder die Kombination aus einer rekombinanten Fettsäurereduktase und aus einer die Fettsäurereduktase phosphopantheteinylierenden Phosphopantheteinyl-Transferase exprimiert, und der zusätzlich eine Transaminase exprimiert, wobei die Phosphopantheteinyl-Transferase und/oder Transaminase bevorzugt rekombinant ist; sowie ein Verfahren zur Umsetzung einer Carbonsäure oder Dicarbonsäure oder eines Monoesters davon zu einem Amin oder Diamin, umfassend die Schritte Kontaktieren der Carbonsäure oder Dicarbonsäure oder des Monoesters davon mit einer phosphopantheteinylierten Fettsäurereduktase oder einer α-Dioxygenase und Kontaktieren des Produktes mit einer Transaminase.

Polyamide sind eine Klasse von Polymeren, die durch sich wiederholende Amidgruppen gekennzeichnet sind. Der Begriff "Polyamid" betrifft im Unterschied zu den chemisch verwandten Proteinen gewöhnlich synthetische, im Handel erhältliche, thermoplastische Kunststoffe. Polyamide werden von primären Aminen oder von sekundären Aminen abgeleitet, die herkömmlich über gecrackte Kohlenwasserstoffe gewonnen werden. Es können jedoch auch Derivate, genauer Aminocarbonsäuren, Lactame und Diamine, zur Polymerherstellung verwendet werden. Von Interesse sind weiterhin kurzkettige, gasförmige Alkane als Edukte, die ausgehend von nachwachsenden Rohstoffen mit biotechnologischen Verfahren gewonnen werden können.

Die konventionelle chemisch-technische Erzeugung von derartigen Aminen und Diaminen ist von der Versorgung mit fossilen Rohstoffen abhängig, ineffizient, und es fallen dabei große Mengen unerwünschter Nebenprodukte an, in manchen Schritten der Synthese bis zu 80 %. Ein Beispiel für einen solchen Prozess stellt die Herstellung von Laurinlactam dar. Konventionell erfolgt diese über ein vielstufiges Verfahren, das nicht nur eine geringe Ausbeute ergibt, sondern gleichzeitig die Bereitstellung einer aufwändigen Infrastruktur erfordert.

Angesichts dieser Nachteile wurden Verfahren entwickelt, um Amine und Diamine unter Verwendung von Biokatalysatoren ausgehend von nachwachsenden Rohstoffen zu gewinnen. Als nachwachsende Rohstoffe kommen insbesondere Quellen für Fettsäuren in Frage, die in Form von Rapsöl, Kugeldistelöl, Palmkernöl, Kokosnussöl, Sonnenblumenkernöl und ähnlichen Naturprodukten aus einer Vielzahl von biologischen Quellen, insbesondere aus Pflanzen, gewonnen werden können.

PCT/EP 2008/067447 beschreibt ein biotechnologisches System zur Herstellung chemisch verwandter Produkte, genauer ω-Aminocarbonsäuren, unter Verwendung einer Zelle, die eine Reihe von geeigneten enzymatischen Aktivitäten aufweist und in der Lage ist, Carbonsäuren zu entsprechenden ω-Aminocarbonsäure umzuwandeln. Das Verfahren umfasst eine Kaskade enzymatisch katalysierter Reaktionen, insbesondere die Oxidation einer Fettsäure am endständigen Kohlenstoffatom bis zum Aldehyd und die anschließende Aminierung unter Verwendung einer Transaminase und einer Aminosäure als Amindonor, die über eine Aminosäuredehydrogenase regeneriert werden kann.

Ein bekannter Nachteil des dabei verwendeten AlkBGT-Oxidasesystems aus *Pseudomonas putida* GPO1 besteht jedoch darin, dass es nicht in der Lage ist, eine selektive Oxidation von aliphatischen Alkanen zu primären Alkoholen zu leisten. Vielmehr treten eine Vielzahl von Oxidationsprodukten auf; insbesondere erhöht sich der Anteil an höher oxidierten Produkten wie dem entsprechenden Aldehyd, Keton oder der entsprechenden Carbonsäure mit zunehmender Reaktionsdauer (C. Grant, J. M. Woodley and F. Baganz (2011), Enzyme and Microbial Technology 48, 480-486), was die Ausbeute an erwünschtem Amin entsprechend verringert.

Das Problem der relativ unselektiven Oxidation ist dadurch verschärft, dass die entstehenden Oxidationsprodukte strukturell sehr ähnlich sind. Dies bedingt, dass es sehr schwierig ist, sie von den erwünschten Oxidationsprodukten effizient und ohne signifikanten Ausbeuteverlust abzutrennen.

Ein weiterer Nachteil dieses Verfahrens besteht darin, dass überoxidierte Nebenprodukte, beispielsweise die Dicarbonsäure der als Edukt eingesetzten Fettsäure, das Recycling von hydrophoben Lösungsmitteln und hydrophoben flüssigen Kationenaustauschern, die gemäß der PCT/EP2011/071491 zur Abtrennung des Produktes aus der wässrigen Reaktionsmischung verwendet werden können, auf Kosten der Effizienz bei der Ressourcennutzung gehen.

In diesem Zusammenhang ist hervorzuheben, dass die Komplexität biotechnologischer Systeme mit einer Kaskade von Reaktionen wie dem in der PCT/EP 2008/067447 beschriebenen, von denen jeweils eine Reaktion von einem bestimmten Enzym katalysiert wird, die Optimierung der Reaktionsbedingungen erschwert. So ist im Falle der grundsätzlich reaktiven ω-Aminofettsäuren als Produkt die Möglichkeit gegeben, dass sie ab einer bestimmten kritischen Konzentration im Inneren der Zelle mit essentiellen Bestandteilen des Organismus reagieren und somit toxisch wirken. Ist das der Fall, so ist die Wachstums- und Synthesefähigkeit des Organismus bis hin zum Absterben der Zelle beeinträchtigt, ohne dass der Entwickler die Toxizität unmittelbar erkennen oder gar einem bestimmten Edukt, Zwischenprodukt oder Produkt zuordnen könnte. Welcher Organismus welche Konzentration an einem chemisch reaktiven Stoff verträgt, ist ebenfalls nicht vorhersehbar.

Auch mit Hinblick auf eine zu verbessernde Produktausbeute und eine zu verringernde Entstehung von Nebenprodukten kann der Fachmann limitierende und entscheidende Faktoren in einem System wie dem in der PCT/EP2008/067447 beschriebenen nicht routinemäßig identifizieren. Ist die Ausbeute an Produkt zu niedrig, so kann dies daran liegen, dass eines der Enzyme in einer zu niedrigen Konzentration vorhanden ist, ohne dass bekannt wäre, um welches der in Frage kommenden Enzyme es sich dabei handelt, d. h. das Edukt wird im vorgesehenen Zeitrahmen oder vor dem Abbau durch konkurrierende Enzyme aufgrund unzureichender Synthesekapazität nicht umgesetzt. Alternativ ist es durchaus möglich, dass ein Enzym zwar nachweisbar in der Zelle in Form eines Polypeptids vorhanden ist, aber gerade in dieser Zelle nicht die für die Aktivität essentielle Faltung aufweist oder ein bis dato unbekannter, für die Aktivität aber essentieller Cofaktor fehlt. Gleichermaßen kann, wie bereits erwähnt, das Stoffwechselprodukt für die Zelle toxisch sein oder abgebaut werden. Schließlich ist mit interferierenden Wechselwirkungen mit endogenen, d. h. natürlich in einer als Ganzzellkatalysator verwendeten Zelle vorhandenen Enzymen zu rechnen.

Es besteht somit Bedarf an Verfahren zur Herstellung von Alkylmonoaminen und -diaminen aus Fettsäuren, bei denen die enzymatisch katalysierten Reaktionen selektiver verlaufen und die Bildung unerwünschter Nebenprodukte minimiert ist.

Vor diesem Hintergrund besteht die der Erfindung zu Grunde liegende Aufgabe darin, ein mit Hinblick auf Ausbeute, Kohlenstoff- und/oder Stickstoffbilanz und/oder Reinheit möglichst effizientes biotechnologisches Verfahren zur Herstellung von Alkylmonoaminen und -diaminen aus Fettsäuren bereitzustellen.

Eine weitere der Erfindung zu Grunde liegende Aufgabe besteht darin, ein mit Hinblick auf Ausbeute, Kohlenstoff- und/oder Stickstoffbilanz, Wiederverwendbarkeit verwendeter Agenzien und/oder Reinheit des Produktes möglichst effizientes biotechnologisches Verfahren zur Herstellung von Alkylmonoaminen und -diaminen aus Fettsäuren bereitzustellen. In diesem Zusammenhang wird unter einer effizienten Kohlenstoff- und/oder Stickstoffbilanz bevorzugt verstanden, dass sich ein möglichst hoher Anteil des zur Umsetzung einer Carbonsäure oder eines Carbonsäureesters in Form von geeigneten Substraten an eine Zelle verfütterten Kohlenstoffs und/oder Stickstoffs im erwünschten Endprodukt wiederfindet, anstatt beispielsweise zu anderen Produkten als den erwünschten umgesetzt zu werden.

Eine weitere der Erfindung zu Grunde liegende Aufgabe besteht darin, die Aufarbeitbarkeit einer mehrphasigen Reaktionsmischung aus der Herstellung von Alkylmonoaminen und - diaminen aus Fettsäuren zu verbessern, besonders mit Hinblick auf die Wiederverwendbarkeit zur Aufarbeitung verwendeter hydrophober Lösungsmittel und flüssiger Kationenaustauscher, sowie mit Hinblick auf die Phasenbildung und -trennung bei einem biphasischen System umfassend eine wässrige Phase, in der die Umsetzung der Carbonsäure oder des Carbonsäureesters abläuft, und eine organische Phase mit organischen Lösungsmitteln und/oder flüssigen Kationenaustauschern.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Das der Erfindung zu Grunde liegende Problem wird in einem ersten Aspekt gelöst durch einen Ganzzellkatalysator, der eine rekombinante α-Dioxygenase oder die Kombination aus einer rekombinanten Fettsäurereduktase und aus einer die Fettsäurereduktase phosphopantheteinylierenden Phosphopantheteinyl-Transferase exprimiert, und der zusätzlich eine Transaminase exprimiert. In einer ersten Ausführungsform des ersten Aspekts wird das Problem durch einen Ganzzellkatalysator gelöst, der zusätzlich eine Aminosäuredehydrogenase exprimiert. In einer zweiten Ausführungsform, die auch eine Ausführungsform der ersten Ausführungsform darstellt, wird das Problem durch einen Ganzzellkatalysator gelöst, der zusätzlich eine Alkanhydroxylase exprimiert. In einer dritten Ausführungsform, die auch eine Ausführungsform der ersten bis zweiten Ausführungsform darstellt, wird das Problem durch einen Ganzzellkatalysator gelöst, der zusätzlich ein Polypeptid der AlkL-Familie exprimiert. In einer vierten Ausführungsform, die auch eine Ausführungsform der zweiten Ausführungsform darstellt, wird das Problem durch einen Ganzzellkatalysator gelöst, der zusätzlich eine Alkoholdehydrogenase exprimiert. In einer fünften Ausführungsform, die auch eine Ausführungsform der ersten bis vierten Ausführungsform darstellt, wird das Problem durch einen Ganzzellkatalysator gelöst, wobei die Aktivität wenigstens eines an der β-Oxidation beteiligten Enzyms gegenüber dem Wildtyp des Ganzzellkatalysators verringert ist.

In einer sechsten Ausführungsform, die auch eine Ausführungsform der ersten bis fünften Ausführungsform darstellt, wird das Problem durch einen Ganzzellkatalysator gelöst, wobei die Aktivität von BioH oder einer Variante davon gegenüber dem Wildtyp des Ganzzellkatalysators verringert oder erhöht ist.

In einer siebten Ausführungsform, die auch eine Ausführungsform der ersten bis sechsten Ausführungsform darstellt, wird das Problem durch einen Ganzzellkatalysator gelöst, wobei die Aktivität von FadL oder einer Variante davon gegenüber dem Wildtyp des Ganzzellkatalysators erhöht ist.

In einem zweiten Aspekt wird das der Erfindung zu Grunde liegende Problem gelöst durch ein Verfahren zur Umsetzung einer Carbonsäure oder Dicarbonsäure oder eines Monoesters davon zu einem Amin oder Diamin, umfassend die Schritte
a) Bereitstellen einer Carbonsäure oder Dicarbonsäure oder eines Monoesters davon, für den Fall, dass es sich um eine Dicarbonsäure handelt,
b) Kontaktieren des Carbonsäure oder Dicarbonsäure oder des Monoesters davon mit einer phosphopantheteinylierten Fettsäurereduktase oder einer α-Dioxygenase unter Bildung eines Aldehydproduktes, und
c) Kontaktieren des Produktes aus Schritt b) mit einer Transaminase.

In einem zweiten Aspekt wird das der Erfindung zu Grunde liegende Problem gelöst durch ein Verfahren, wobei bei Schritt c) eine Aminosäuredehydrogenase anwesend ist.

In einer ersten Ausführungsform des zweiten Aspekts wird das Problem gelöst durch ein Verfahren, wobei wenigstens ein Enzym aus der Gruppe umfassend phosphopantheteinylierte Fettsäurereduktase, α-Dioxygenase, Transaminase, Aminosäuredehydrogenase und Alkanhydroxylase, in Form eines Ganzzellkatalysators dem ersten Aspekt der vorliegenden Erfindung bereitgestellt werden.

In einer zweiten Ausführungsform, die auch eine Ausführungsform der ersten Ausführungsform darstellt, wird das Problem gelöst durch ein Verfahren, wobei es sich bei der Carbonsäure oder Dicarbonsäure oder dem Monoester davon um eine Verbindung der Formel (I)

R¹-A-COOR² (I)

handelt, wobei R¹ aus der Gruppe ausgewählt ist, die -H und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoffatomen darstellt.

In einer dritten Ausführungsform, die auch eine Ausführungsform der ersten bis zweiten Ausführungsform darstellt, wird das Problem gelöst durch ein Verfahren, wobei A die Formel - (CH₂)ₙ - aufweist, wobei n wenigstens 4, bevorzugt wenigstens 10 ist.

In einem dritten Aspekt wird das der Erfindung zu Grunde liegende Problem gelöst durch eine Verwendung des Ganzzellkatalysators nach dem ersten Aspekt oder des Verfahrens nach dem zweiten Aspekt zur Aminierung einer Carbonsäure oder Dicarbonsäure oder eines Monoesters davon.

In einem vierten Aspekt wird das der Erfindung zu Grunde liegende Problem gelöst durch eine Reaktionsmischung umfassend den Ganzzellkatalysator nach dem ersten Aspekt in wässriger Lösung sowie eine Fettsäure, ω-Hydroxy-Fettsäure, ω-Oxo-Fettsäure oder einen Monoester davon der Formel (I)

R¹-A-COOR² (I),

wobei R¹ aus der Gruppe ausgewählt ist, die -H, -CHO, -OH und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoffatomen darstellt.
Die vorliegende Erfindung beruht auf der Erkenntnis der Erfinder, dass eine funktionell rekombinante Fettsäurereduktase oder α-Dioxygenase in einem Ganzzellkatalysator, der zur Herstellung von Aminen und Diaminen aus Fettsäuren verwendet wird und eine entsprechende Enzymausstattung aufweist, überraschend die Ausbeute an Aminen und Diaminen erhöht.

Weiterhin beruht die vorliegende Erfindung auf der Erkenntnis der Erfinder, dass eine funktionell rekombinante Fettsäurereduktase oder α-Dioxygenase in einem Ganzzellkatalysator, der zur Herstellung von Aminen und Diaminen aus Fettsäuren verwendet wird und eine entsprechende Enzymausstattung aufweist, überraschend die Konzentration störender Nebenprodukte, insbesondere von überoxidierten Fettsäuren in Form von Dicarbonsäuren und Estern davon, im anfallenden Produkt verringert.

Weiterhin beruht die vorliegende Erfindung auf der Erkenntnis der Erfinder, dass eine funktionell rekombinante Fettsäurereduktase oder α-Dioxygenase in einem Ganzzellkatalysator, der zur Herstellung von Aminen und Diaminen Fettsäuren verwendet wird und eine entsprechende Enzymausstattung aufweist, die Reinheit und Wiederverwendbarkeit von flüssigen Kationenaustauschern wie Ölsäure verbessert, die zur Entfernung des Amins und Diamins aus einem Fermentationslösung umfassend den Ganzzellkatalysator verwendet werden.

Die vorliegende Erfindung betrifft ein verbessertes Verfahren zur Umsetzung einer Carbonsäure oder Dicarbonsäure oder eines Monoesters davon zu einem Amin oder Diamin, das sich dadurch auszeichnet, dass neben den Enzymen, die die Überführung der Fettsäure über ihre verschiedenen Oxidationsstufen zum Amin katalysieren, auch eine Fettsäurereduktase oder α-Dioxygenase anwesend ist, bevorzugt, wenn ein Ganzzellkatalysator zur Durchführung des Verfahrens eingesetzt wird. In einer bevorzugten Ausführungsform wird unter dem Begriff "Fettsäurereduktase", wie hierin verwendet, ein Enzym verstanden, das die Umwandlung einer ω-Carboxysäure, auch als Dicarbonsäure oder ω-Carboxy-Fettsäure bezeichnet, zur entsprechenden ω-Oxofettsäure unter Verbrauch von ATP und NAD(P)H katalysiert. Im Stand der Technik, beispielsweise in der WO/2010/135624, sind Fettsäurereduktasen zur Herstellung von ω-Hydroxyfettsäuren beschrieben, nicht jedoch als Teil eines Systems zu Herstellung von ω-Aminofettsäuren. In einer noch bevorzugteren Ausführungsform ist die Fettsäurereduktase aus der Gruppe von Fettsäurereduktasen ausgewählt, die die Aminosäuresequenzen YP_887275.1, ZP_11001941.1, ZP_06852401.1, NP_959974.1, YP_001070587.1, ZP_05217435.1, YP_882653.1, YP_639435.1, ZP_10800193.1, YP_006452763.1, YP_006730440.1, ZP_11196216.1, YP_005349252.1, ZP_05224908.1, YP_005338837.1, YP_006307000.1, YP_005343991.1, ZP_11001942.1, ZP_09979565.1, YP_005003162.1, YP_953393.1, YP_001850422.1, ZP_11011489.1, ZP_12689264.1, YP_905678.1, ZP_09976919.1, YP_004746059.1, NP_217106.1, YP_004525443.1, NP_337166.1, ZP_09685823.1, YP_978699.1, ZP_06437984.1, ZP_06514086.1, NP_856267.1, CAA19077.1, NP_301424.1, ZP_06522140.1, ZP_06518098.1, ZP_11008938.1, ZP_07432374.2, AAR91681.1, YP_006808747.1, YP_001851230.1, ZP_15327751.1, ZP_15455857.1, ZP_12874284.1, ZP_15332534.1, ZP_15512956.1, ZP_14244106.1, ZP_15470899.1, ZP_11439367.1, YP_001703694.1, ZP_15446742.1, YP_006808978.1, ZP_07964926.1, YP_006521379.1, ZP_10796908.1, ZP_15512957.1, ZP_12874283.1, YP_005350955.1, ZP_14243341.1, YP_001705436.1, ZP_15329649.1, YP_006522325.1, YP_006732197.1, YP_003658971.1, ZP_05227804.1, YP_001703695.1, YP_006308707.1, ZP_15342047.1, YP_006521380.1, ZP_15327752.1, YP_005340557.1, ZP_11439578.1, ZP_15392943.1, ZP_15514789.1, ZP_12996178.1, ZP_09412214.1, ZP_06849686.1, YP_889972.1, YP_006570321.1, ZP_15375693.1, YP_006308219.1, YP_006521600.1, YP_005340029.1, YP_005350457.1, ZP_11439836.1, ZP_12994664.1, ZP_14240588.1, ZP_14236860.1, ZP_09410830.1, YP_006731697.1, YP_005264225.1, YP_001704097.1, ZP_15328186.1, ZP_09402885.1, ZP_12690463.1, AFO59871.1, ZP_07966879.1, YP_118225.1, YP_001828302.1, YP_006566873.1, YP_003660169.1, ZP_15337407.1, ZP_08240521.1, ZP_10456477.1, YP_001537947.1, YP_004016539.1, ZP_07664024.1, ZP_14244107.1, ZP_09794557.1, ZP_09274211.1, ZP_05224899.1, ZP_15484175.1, AAA17105.1, ZP_11437924.1, ZP_15446621.1, YP_003646340.1, ZP_15382134.1, ZP_14237669.1, ZP_09165547.1, YP_004019203.1, ZP_14240225.1, YP_001220863.1, CBA74242.1, ZP_12994240.1, EIE27140.1, ZP_15354547.1, ZP_15432557.1, ZP_15500132.1, ZP_15478632.1, ZP_06846978.1, AAA17108.1, ZP_15333767.1, ZP_05217205.1, AAD44234.1, YP_005348984.1, YP_006306749.1, ZP_05224611.1, YP_005343772.1, YP_006730188.1, YP_882425.1, ZP_10799956.1, ZP_05045132.1, NP_960176.1, ZP_12398880.1, ZP_11192735.1, ZP_11440091.1, ZP_05217203.1, ZP_06846979.1, ZP_10800936.1, ZP_06523596.1, YP_882421.1, YP_006306748.1, YP_006522017.1, ZP_15432556.1, ZP_15354095.1, ZP_05227781.1, ZP_09684639.1, YP_006730187.1, YP_005343770.1, YP_005338616.1, YP_005348983.1, ZP_15472813.1, ZP_15457007.1, ZP_15421152.1, ZP_15488933.1, ZP_14240030.1, YP_001704825.1, ZP_15328982.1, YP_005911512.1, ZP_09411638.1, ZP_12876400.1, ZP_12995435.1, ZP_07667680.1, YP_001281387.1, EIE21044.1, ZP_15375054.1, NP_334518.1, 4DQV_A, ZP_06435375.1, YP_003030020.1, YP_976237.1, ZP_04926822.1, YP_004998149.1, YP_004743589.1, YP_005907921.1, NP_214615.1, YP_001286047.1, ZP_06515541.1, ZP_05139482.1, YP_888016.1, ZP_06452908.1, ZP_06519578.1, YP_004721827.1, CAJ77696.1, ZP_09680854.1, ZP_09686453.1, YP_884815.1, YP_884815.1,
CAB55600.1, ZP_09081423.1, YP_006521568.1, ZP_11440626.1, ZP_15513309.1, ZP_09410778.1, ZP_15374248.1, ZP_15405954.1, YP_001704047.1, ZP_14236911.1, ZP_12873916.1, ZP_14242094.1, ZP_12994610.1, ZP_07664023.1, ZP_15446620.1, ZP_15484174.1, ZP_14240245.1, YP_005358845.1 und XP_002669159.1,
insbesondere YP_006731697.1, ZP_09839660.1, YP_001704097.1, YP_889972.1, ZP_05045132.1, ZP_09794557.1, ZP_08240521.1, NP_959974.1, ZP_10456477.1, YP_118225.1, NP_217106, YP_905678.1, YP_887275.1, ZP_11001941.1, YP_953393.1 und YP_005349252.1 Varianten davon umfassen.

Bei Fettsäurereduktasen handelt es sich um eine Gruppe von Enzymen, die für ihre Aktivität eine Phosphopantheteinylierung, d. h. die kovalente Befestigung eines Phosphopantheteinyl-Cofaktors am Enzym, benötigen. Dementsprechend ist die erfindungsgemäß verwendete Fettsäurereduktase phosphopantheteinyliert, und eine die Fettsäurereduktase exprimierender Ganzzellkatalysator exprimiert, entweder als Teil seiner Ausstattung endogen exprimierter Enzyme oder in rekombinanter Form, eine die Fettsäurereduktase phosphopantheteinylierende Phosphopantheteinyl-Transferase. In einer bevorzugten Ausführungsform wird unter dem Begriff "Phosphopantheteinyl-Transferase", wie hierin verwendet, ein Enzym verstanden, dass einen Phosphopantheteinyl-Rest von einem Phosphopantheteinyl-CoA auf ein Enzym, bevorzugt an der Fettsäurereduktase, überträgt. In einer besonders bevorzugten Ausführungsform ist die Phosphopantheteinyl-Transferase aus der Gruppe von Phosphopantheteinyl-Transferasen ausgewählt, die die Aminosäuresequenzen ABI83656.1, YP_006811024.1, YP_120266.1, YP_005265173.1, YP_004006671.1, ZP_08152482.1, ZP_11104141.1, ZP_14482198.1, YP_706581.1, ZP_10002626.1, ZP_09308410.1, YP_002783881.1, ZP_18276502.1, ZP_09271851.1, ZP_08204640.1, YP_002766085.1, ZP_09788717.1, ZP_09799863.1, ZP_10961877.1, YP_003273299.1, GAB86168.1, YP_006668875.1, ZP_08766535.1, ZP_09793386.1, ZP_09212827.1, ZP_09276344.1, ZP_09213870.1, ZP_09081490.1, ZP_10947586.1, YP_003658841.1, ZP_06852853.1, YP_953148.1, ZP_11011170.1, YP_639258.1, YP_886985.1, ZP_11194383.1, ZP_09681094.1, ZP_06455719.1, NP_337369.1, YP_004077819.1, NP_217310.1, YP_006452521.1, YP_005339056.1, ZP_05226335.1, ZP_07965127.1, ZP_07419314.2, NP_302077.1, YP_005003342.1, YP_005349465.1, ZP_10800435.1, ZP_06564430.1, YP_882860.1, YP_001135287.1, YP_001850220.1, ZP_05217634.1, YP_003646683.1, YP_004746246.1, ZP_15327906.1, ZP_09979035.1, YP_001703848.1, YP_906028.1, ZP_15395499.1, ZP_11438833.1, ZP_11005955.1, ZP_09410582.1, NP_961833.1, YP_001106197.1, ZP_14237113.1, YP_004085491.1, YP_003835595.1, ZP_12994399.1, YP_004523804.1, ZP_12690887.1, YP_003339468.1, ZP_06589331.1, YP_004801334.1, ZP_09974565.1, ZP_04608379.1, ZP_13037142.1, YP_712537.1, ZP_11236665.1, NP_630748.1, ZP_06527138.1, YP_003835167.1, CCH33620.1, ZP_10309401.1, ZP_08881396.1, YP_003102953.1, YP_003487252.1, ZP_08881565.1, YP_006263961.1, NP_822924.1, YP_004914569.1, ZP_09400366.1, AFV71333.1, ZP_07309518.1, ZP_09172171.1, ZP_06710898.1, CAN89630.1, ZP_06921116.1, ZP_08804003.1, ZP_19189663.1, ZP_10545589.1, YP_006248725.1, ZP_10455557.1, YP_004015869.1, ZP_08801530.1, ZP_10550999.1, YP_004492879.1, ZP_09958730.1, ZP_08286666.1, ZP_11212856.1, AAL15597.1, AAZ94407.1, ZP_19188802.1, AFF18625.1, ZP_06575404.1, AAK06801.1, ADC79635.1, YP_004080528.1, YP_004921314.1, ACY01405.1, YP_004584022.1, YP_003114157.1, YP_003203177.1, AFB69911.1, YP_006876460.1, ZP_08024798.1, YP_006269867.1, YP_006881814.1, CCK26150.1, ZP_07307765.1, ZP_07315112.1, YP_005466392.1, NP_824081.1, YP_003493882.1, ZP_06412387.1, ZP_10068239.1, ZP_08234258.1, YP_001822177.1, ZP_03979107.1, ZP_07979043.1, BAA22407.1, ZP_09402950.1, YP_003112617.1, NP_738483.1, YP_480609.1, EKX90208.1, BAE93744.1, BAB69186.1, ZP_04713061.1, YP_006881735.1, ZP_07274901.1, ZP_11379052.1, ZP_06581115.1, YP_006437406.1, ZP_12871839.1, NP_601186.1, ZP_08451808.1, YP_005057339.1, YP_005303909.1, ZP_07090824.1, YP_003783676.1, YP_004630011.1, ZP_06588772.1, AAX98203.1, AFK80329.1, ZP_08124665.1, ZP_03710365.1, AAB17877.1, ZP_07403633.1, ZP_11268660.1, ZP_07288841.1, ABV83217.1, ZP_16178576.1, AAG43513.1, ZP_09155938.1, YP_004605750.1, ZP_03918977.1, AAF71762.1, ZP_05007864.1, ZP_06836265.1, ZP_03934882.1, YP_001508477.1, ZP_06043756.1, ZP_05366306.1, YP_002835056.1, ZP_03933464.1, ZP_07469321.1, ZP_07713507.1, YP_005160553.1, NP_939820.1, AAU93794.1, ZP_14659796.1, ZP_14383679.1, YP_005058606.1, YP_001221073.1, ZP_08231568.1, YP_250920.1, ZP_11383249.1, YP_003916320.1, ZP_08681170.1, YP_001800249.1, YP_001157632.1, YP_166099.1, ZP_10088015.1, YP_004760065.1, ZP_07947675.1, YP_001603066.1, YP_003812683.1, YP_004403402.1, ZP_08292153.1, ZP_09471260.1, YP_004018108.1, ZP_05115352.1, AAD13565.1, ZP_09295321.1, YP_001535629.1, ZP_04607273.1, YP_006561753.1, ZP_00960958.1, YP_006571985.1, ZP_08862188.1, YP_002906426.1, CCK30433.1, ZP_13042493.1, ZP_09090153.1, YP_614397.1, ZP_11163860.1, YP_003983492.1, YP_004080668.1, ZP_09420475.1, ZP_05914565.1, ZP_01101149.1, ZP_14743088.1, YP_001239694.1, ZP_09127532.1, YP_003833873.1, ZP_08516197.1, ZP_10160483.1, ZP_01987188.1, ZP_01755304.1, ZP_08825027.1, ZP_05077116.1, YP_001444606.1, ZP_03392800.1, ZP_01057781.1, AFB69889.1, ZP_08815097.1 und AAO17175.1 und Varianten davon umfassen. In einer besonders bevorzugten Ausführungsform handelt es sich dabei um die Phosphopantheteinyl-Transferase mit dem Datenbankcode ABI83656.1 oder eine Variante davon.

Alternativ oder zusätzlich zur Kombination aus Fettsäurereduktase und Phosphopantheteinyl-Transferase kann der Ganzzellkatalysator auch eine α-Dioxygenase aufweisen. In einer bevorzugten Ausführungsform wird unter dem Begriff "α-Dioxygenase", wie hierin verwendet, ein Enzym verstanden, dass die Umwandlung einer Carbonsäure und/oder Dicarbonsäure oder eines Monoesters davon unter Verbrauch eines Moleküls Sauerstoff und unter Abspaltung eines Kohlendioxid-Moleküls zu einer am endständigen ω-Kohlenstoffatom gegenüber der als Edukt eingesetzten Carbonsäure und/oder Dicarbonsäure oder eines Monoesters davon um ein Kohlenstoffatom verkürzten, am endständigen ω-Kohlenstoffatom eine Aldehydgruppe tragenden Carbonsäure und/oder Dicarbonsäure oder eines Monoesters davon katalysiert. In einer besonders bevorzugten Ausführungsform ist die α-Dioxygenase aus der Gruppe von α-Dioxygenasen ausgewählt, die die Aminosäuresequenzen NP_001066718.1, EAY82977.1, BAH79993.1, ABG22011.1, BAJ90503.1, AFD04418.1, AFD04417.1, BAJ87736.1, AFW75180.1, ABG22012.1, XP_002311389.1, CAH05011.1, XP_002279884.1, CBI34957.3, AAG59584.1, NP_001234414.1, NP_001234410.1, XP_003553942.1, XP_002275161.1, XP_003553937.1, CBI34960.3, CAA07589.1, XP_003543402.1, XP_002517402.1, XP_002882184.1, NP_186791.1, AAK85133.1, CAN77070.1, XP_002529555.1, CAH64542.1, NP_001234061.1, XP_002281357.1, ADM21465.1, XP_002318527.1, NP_177509.1, CAN74266.1, XP_002888940.1, NP_001185393.1, XP_003631072.1, BAJ33800.1, XP_002517377.1, XP_003530944.1, BAJ34623.1, ABG22013.1, ABP02610.1, XP_001773135.1, XP_002960339.1, ABK95279.1, ABD73303.1, ABD73304.1, YP_001805721.1, ZP_08971815.1, ZP_08430366.1, YP_823013.1, ZP_05026427.1, ZP_11003953.1, YP_007064484.1, YP_007113008.1, YP_633369.1, ZP_18906570.1, ZP_09251410.1, ZP_10050808.1, ZP_01306662.1, YP_001516886.1, ZP_05042862.1, AAC49625.1, ZP_09648375.1, ZP_09792714.1, ZP_09788527.1, XP_001728273.1, AAC83355.1, YP_890542.1, ZP_11000891.1, XP_002605323.1, EGO58341.1, YP_006249145.1, YP_001507004.1, YP_001704637.1, ZP_12876141.1, ZP_11150830.1, ZP_14236257.1, ZP_09411385.1, ZP_14243118.1, EKD16664.1, ZP_15416799.1, ZP_15338016.1, ZP_10080295.1, ZP_11438929.1, ZP_12995210.1, ZP_10946648.1, YP_003409541.1, XP_001637870.1, YP_005451221.1, XP_001212758.1, ZP_07290489.1, ZP_05781329.1, ZP_19187748.1, ZP_06574534.1, XP_002605322.1, NP_822950.1, YP_006366425.1, EJP63377.1, EKD21217.1, XP_001795927.1, XP_003042615.1, ZP_06566152.1, EGU88116.1, EFY94417.1, XP_388327.1, EKJ68934.1, ZP_07290463.1, CCC10458.1, YP_001107201.1, XP_003348248.1, T49753, CAD31840.1, XP_001229975.1, CBN77040.1, YP_004813753.1, XP_002513273.1, XP_001627136.1, AFG52858.1, AFG52857.1, AEW08450.1, NP_841291.1, YP_004512343.1, ACG75701.1 und ZP_03500906.1 und Varianten davon umfassen. In einer besonders bevorzugten Ausführungsform handelt es sich um die α-Dioxygenase mit dem Datenbankcode NP_001066718.1 oder eine Variante davon.

Neben der α-Dioxygenase oder der Kombination aus Fettsäurereduktase und der Phosphopantheteinyl-Transferase weist der erfindungsgemäße Ganzzellkatalysator notwendigerweise eine Transaminase auf, die endständige Aldehydgruppen aminiert. In einer bevorzugten Ausführungsform wird unter dem Begriff "Transaminase", wie hierin verwendet, ein Enzym verstanden, das die Übertragung von α-Aminogruppen von einem Donor-, bevorzugt einer Aminosäure, auf ein Akzeptormolekül, bevorzugt eine α-Ketocarbonsäure, katalysiert. In einer besonders bevorzugten Ausführungsform ist die Transaminase aus der Gruppe von Transaminasen ausgewählt, die die Aminosäuresequenzen 3HMU_A, AAD41041.1, AAK15486.1, ABE03917.1, ADR60699.1, ADR61066.1, ADR62525.1, AEL07495.1, CAZ86955.1, EFW82310.1, EFW87681.1, EGC99983.1, EGD03176.1, EGE58369.1, EGH06681.1, EGH08331.1, EGH24301.1, EGH32343.1, EGH46412.1, EGH55033.1, EGH62152.1, EGH67339.1, EGH70821.1, EGH71404.1, EGH78772.1, EGH85312.1, EGH97105.1, EGP57596.1, NP_102850.1, NP_106560.1, NP_248912.1, NP_248990.1, NP_354026.2, NP_421926.1, NP_637699.1, NP_642792.1, NP_744329.1, NP_744732.1, NP_747283.1, NP_795039.1, NP_901695.1 (, XP_002943905.1, YP_001021095.1, YP_001059677.1, YP_001061726.1, YP_001066961.1, YP_001074671.1, YP_001120907.1, YP_001140117.1, YP_001170616.1, YP_001185848.1, YP_001188121.1, YP_001233688.1, YP_001268866.1, YP_001270391.1, YP_001345703.1, YP_001412573.1, YP_001417624.1, YP_001526058.1, YP_001579295.1, YP_001581170.1, YP_001668026.1, YP_001669478.1, YP_001671460.1, YP_001685569.1, YP_001747156.1, YP_001749732.1, YP_001765463.1, YP_001766294.1, YP_001790770.1, YP_001808775.1, YP_001809596.1, YP_001859758.1, YP_001888405.1, YP_001903233.1, YP_001977571.1, YP_002229759.1, YP_002231363.1, YP_002280472.1, YP_002297678.1, YP_002543874.1, YP_002549011.1, YP_002796201.1, YP_002801960.1, YP_002875335.1, YP_002897523.1, YP_002912290.1, YP_002974935.1, YP_003060891.1, YP_003264235.1, YP_003552364.1, YP_003578319.1, YP_003591946.1, YP_003607814.1, YP_003641922.1, YP_003674025.1, YP_003692877.1, YP_003755112.1, YP_003896973.1, YP_003907026.1, YP_003912421.1, YP_004086766.1, YP_004142571.1, YP_004147141.1, YP_004228105.1, YP_004278247.1, YP_004305252.1, YP_004356916.1, YP_004361407.1, YP_004378186.1, YP_004379856.1, YP_004390782.1, YP_004472442.1, YP_004590892.1, YP_004612414.1, YP_004676537.1, YP_004693233.1, YP_004701580.1, YP_004701637.1, YP_004704442.1, YP_108931.1, YP_110490.1, YP_168667.1, YP_237931.1, YP_260624.1, YP_262985.1, YP_271307.1, YP_276987.1, YP_334171.1, YP_337172.1, YP_350660.1, YP_351134.1, YP_364386.1, YP_366340.1, YP_369710.1, YP_370582.1, YP_426342.1, YP_440141.1, YP_442361.1, YP_468848.1, YP_521636.1, YP_554363.1, YP_608454.1, YP_610700.1, YP_614980.1, YP_622254.1, YP_625753.1, YP_680590.1, YP_751687.1, YP_767071.1, YP_774090.1, YP_774932.1, YP_788372.1, YP_858562.1, YP_928515.1, YP_983084.1, YP_995622.1, ZP_00948889.1, ZP_00954344.1, ZP_00959736.1, ZP_00998881.1, ZP_01011725.1, ZP_01037109.1, ZP_01058030.1, ZP_01076707.1, ZP_01103959.1, ZP_01167926.1, ZP_01224713.1, ZP_01442907.1, ZP_01446892.1, ZP_01550953.1, ZP_01625518.1, ZP_01745731.1, ZP_01750280.1, ZP_01754305.1, ZP_01763880.1, ZP_01769626.1, ZP_01865961.1, ZP_01881393.1, ZP_01901558.1, ZP_02145337.1, ZP_02151268.1, ZP_02152332.1, ZP_02167267.1, ZP_02190082.1, ZP_02242934.1, ZP_02360937.1, ZP_02367056.1, ZP_02385477.1, ZP_02456487.1, ZP_02883670.1, ZP_03263915.1, ZP_03263990.1, ZP_03400081.1, ZP_03452573.1, ZP_03456092.1, ZP_03517291.1, ZP_03529055.1, ZP_03571515.1, ZP_03572809.1, ZP_03587785.1, ZP_03588560.1, ZP_03697266.1, ZP_03697962.1, ZP_04521092.1, ZP_04590693.1, ZP_04890914.1, ZP_04891982.1, ZP_04893793.1, ZP_04902131.1, ZP_04905327.1, ZP_04941068.1, ZP_04944536.1, ZP_04945255.1, ZP_04959332.1, ZP_04964181.1, ZP_05053721.1, ZP_05063588.1, ZP_05073059.1, ZP_05077806.1, ZP_05082750.1, ZP_05091128.1, ZP_05095488.1, ZP_05101701.1, ZP_05116783.1, ZP_05121836.1, ZP_05127756.1, ZP_05637806.1, ZP_05742087.1, ZP_05783548.1, ZP_05786246.1, ZP_05843149.1, ZP_05945960.1, ZP_06459045.1, ZP_06487195.1, ZP_06492453.1, ZP_06493162.1, ZP_06703644.1, ZP_06731146.1, ZP_06839371.1, ZP_07007312.1, ZP_07266194.1, ZP_07374050.1, ZP_07662787.1, ZP_07778196.1, ZP_07797983.1, ZP_08099459.1, ZP_08138203.1, ZP_08141719.1, ZP_08142973.1, ZP_08177102.1, ZP_08185821.1, ZP_08186468.1, ZP_08208888.1, ZP_08266590.1, ZP_08402041.1, ZP_08406891.1, ZP_08522175.1, ZP_08527488.1, ZP_08631252.1, ZP_08636687 und Varianten davon umfassen.

Bei der erfindungsgemäß verwendeten Fettsäurereduktase und bevorzugt auch bei anderen erfindungsgemäß verwendeten Enzymen handelt es sich um rekombinante Enzyme. In einer bevorzugten Ausführungsform wird unter dem Begriff "rekombinant", wie hierin verwendet, verstanden, dass das für das entsprechende Enzym kodierende Nukleinsäuremolekül in der natürlichen Zelle nicht vorkommt und/oder es unter Verwendung von gentechnischen Methoden hergestellt wurde. In einer bevorzugten Ausführungsform spricht man von einem rekombinanten Protein, wenn das entsprechende Polypeptid von einer rekombinanten Nukleinsäure kodiert ist. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook/Fritsch/Maniatis (1989): Molecular Cloning: A Laboratory Manual, Cold Spring Harbor Laboratory Press, 2nd edition, beschriebenen. Rekombinante Enzyme werden bevorzugt überexprimiert, beispielsweise unter Verwendung von pET- oder pGEX-Vektor-Systemen, die dem Fachmann bekannt sind.

Mit Hinblick auf die Wahl des Organismus unterliegt der erfindungsgemäß einsetzbare Ganzzellkatalysator keinen Einschränkungen, sofern er kultivierbar, stabil und ggf. gentechnisch einführbaren Modifikationen, z. B. Verfahren zur Abschwächung von Enzymaktivitäten, beispielsweise Knock outs, zugänglich ist. So kann es sich gleichermaßen um eine prokaryontische oder eukaryontische Zelle handeln. Im Falle einer eukaryontischen Zelle sind unizelluläre Eukaryonten besonders bevorzugt, besonders Hefen wie *Saccharomyces cerevisiae, Candida tropicalis, Candida albicans* und *Pichia pastoris.* Im Falle von prokaryontischen Zellen kann es sich beispielsweise um ein Bakterium handeln, das aus der Gruppe ausgewählt ist, die *Magnetococcus, Mariprofundus, Acetobacter, Acetobacterium, Acidiphilium, Afipia, Ahrensia, Asticcacaulis, Aurantimonas, Azorhizobium, Azospirillum, Bacillus, Bartonella, tribocorum, Beijerinckia, Bradyrhizobium, Brevundimonas, subvibrioides, Brucella, Caulobacter, Chelativorans, Citreicella, Citromicrobium, Clostridium, Corynebacterium, Dinoroseobacter, Erythrobacter, Fulvimarina, Gluconacetobacter, Granulibacter, Hirschia, Hoeflea, Hyphomicrobium, Hyphomonas, Ketogulonicigenium, Labrenzia, Loktanella, Magnetospirillum, Maricaulis, Maritimibacter, Mesorhizobium, Methylobacterium, Methylocystis, Methylosinus, Nitrobacter, Novosphingobium, Oceanibulbus, Oceanicaulis, Oceanicola, Ochrobactrum, Octadecabacter, Oligotropha, Paracoccus, Parvibaculum, Parvularcula, Pelagibaca, Phaeobacter, Phenylobacterium, Polymorphum, Pseudovibrio, Rhodobacter, Rhodomicrobium, Rhodopseudomonas, Rhodospirillum, Roseibium, Roseobacter, Roseomonas, Roseovarius, Ruegeria, Sagittula, Silicibacter, Sphingobium, Sphingomonas, Sphingopyxis, Starkeya, Sulfitobacter, Thalassiobium, Xanthobacter, Zymomonas, Agrobacterium, Rhizobium, Sinorhizobium, Anaplasma, Ehrlichia, Neorickettsia, Orientia, Rickettsia, Wolbachia, Bordetella, Burkholderia, Cupriavidus, Taiwanensis, Lautropia, Limnobacter, Polynucleobacter, Ralstonia, Chromobacterium, Eikenella, corrodens, Basfia, Kingella, Laribacter, Lutiella, Neisseria, Simonsiella, Achromobacter, Acidovorax, Alicycliphilus, Aromatoleum, Azoarcus, Comamonas, Dechloromonas, Delftia, Gallionella, Herbaspirillum, Herminiimonas, Hylemonella, Janthinobacterium, Leptothrix, Methylibium, Methylobacillus, Methylophilales, Methyloversatilis, Methylovorus, Nitrosomonas, Nitrosospira, Oxalobacter, Parasutterella, Polaromonas, Polaromonas, Pusillimonas, Rhodoferax, Rubrivivax, Sideroxydans, Sutterella, wadsworthensis, Taylorella, Thauera, Thiobacillus, Thiomonas, Variovorax, Verminephrobacter, Anaeromyxobacter, Bdellovibrio, bacteriovorus, Bilophila, Desulfarculus, Desulfatibacillum, Desulfobacca, Desulfobacterium, Desulfobulbus, Desulfococcus, Desulfohalobium, Desulfitobacterium, Desulfomicrobium, Desulfonatronospira, Desulfotalea, Desulfovibrio, Desulfuromonas, Geobacter, Haliangium, Hippea, Lawsonia, Myxococcus, Pelobacter, Plesiocystis, Sorangium, Stigmatella, Syntrophobacter, Syntrophus, Arcobacter, Caminibacter, Campylobacter, Helicobacter, Nitratifractor, Nitratiruptor, Sulfuricurvum, Sulfurimonas, Sulfurospirillum, Sulfurovum, Wolinella, Buchnera, Blochmannia, Hamiltonella, Regiella, Riesia, Citrobacter, Cronobacter, Dickeya, Edwardsiella, Enterobacter, Erwinia, Escherichia, Klebsiella, Pantoea, Pectobacterium, Proteus, Providencia, Rahnella, Salmonella, Serratia, Shigella, Sodalis, Wigglesworthia, Glossina, Xenorhabdus, Yersinia, Acidithiobacillus, Acinetobacter, Aeromonas, Alcanivorax, Alkalilimnicola, Allochromatium, Alteromonadales, Alteromonas, Baumannia, Beggiatoa, Bermanella, Carsonella, Ruthia, Vesicomyosocius, Cardiobacterium, Chromohalobacter, Colwellia, Congregibacter, Coxiella, Dichelobacter, Endoriftia, Enhydrobacter, Ferrimonas, Francisella, Glaciecola, Hahella, Halomonas, Halorhodospira, Halothiobacillus, Idiomarina, Kangiella, Legionella, Marinobacter, Marinomonas, Methylobacter, Methylococcus, Methylomicrobium, Methylophaga, Moraxella, Moritella, Neptuniibacter, Nitrococcus, Pseudoalteromonas, Psychrobacter, Psychromonas, Reinekea, Rickettsiella, Saccharophagus, Shewanella, Succinatimonas, Teredinibacter, Thioalkalimicrobium, Thioalkalivibrio, Thiomicrospira, Tolumonas, Vibrionales, Actinobacillus, Aggregatibacter, Gallibacterium, Haemophilus, Histophilus, Mannheimia, Pasteurella, Azotobacter, Cellvibrio, Pseudomonas, Aliivibrio, Grimontia, Photobacterium, Photobacterium, Vibrio, Pseudoxanthomonas, Stenotrophomonas, Xanthomonas, Xylella, Borrelia, Brachyspira, Leptospira, Spirochaeta, Treponema, Hodgkinia, Puniceispirillum, Liberibacter, Pelagibacter, Odyssella, Accumulibacter,* insbesondere *B*. *subtilis, B. megaterium, C. glutamicum, E. coli, Pseudomonas sp., Pseudomonas fluorescens, Pseudomonas putida, Pseudomonas stutzeri,, Acinetobacter* sp., *Burkholderia* sp., *Burkholderia thailandensis,* Cyanobakterien, *Klebsiella* sp., *Klebsiella oxytoca, Salmonella sp., Rhizobium sp.* und *Rhizobium meliloti,* umfasst. In einer besonders bevorzugten Ausführungsform handelt es sich um ein Enterobakterium, am bevorzugtesten um *Escherichia coli.*

Es ist vorteilhaft, wenn der erfindungsgemäße Ganzzellkatalysator neben der Fettsäurereduktase, Phosphopantheteinyl-Transferase und der Transaminase auch eine Alanindehydrogenase aufweist, um das von der Transaminase bei der Aminierung der endständigen Aldehydgruppe verbrauchte Alanin aus anorganischen stickstoffhaltigen Molekülen zu regenerieren. In einer bevorzugten Ausführungsform wird unter dem Begriff "Alanindehydrogenase", wie hierein verwendet, ein Enzym verstanden, das die Umwandlung von L-Alanin unter Verbrauch von Wasser und NAD⁺ zu Pyruvat, Ammoniak und NADH und die umgekehrte Reaktion katalysiert. In einer besonders bevorzugten Ausführungsform ist die Alanindehydrogenase aus der Gruppe von Alanindehydrogenasen ausgewählt, die die Aminosäuresequenz der Alanindehydrogenase aus *Bacillus subtilis* (Datenbankcode L20916), *Rhizobium leguminosarum* (Datenbankcode CP001622), *Vibrio proteolyticus* (Datenbankcode AF070716), *Mycobacterium tuberculosis* (Datenbankcode X63069), *Enterobacter aerogenes* (Datenbankcode AB013821), EGR93259.1, YP_003654745.1, YP_003651439.1, YP_003637111.1, YP_003631815.1, YP_001327051.1, YP_001262560.1, YP_886996.1, YP_882850.1, YP_704410.1, YP_703508.1, ZP_08624689.1, YP_001230376.1, P17557.1, P17556.1, CCB94892.1, CCB73698.1, YP_001168635.1, YP_004668736.1, YP_004569425.1, YP_003513168.1, YP_004561169.1, ZP_08554945.1, YP_400777.1, ZP_08311476.1, ZP_08310170.1, ZP_08267322.1, ZP_08263846.1, ZP_07898723.1, YP_149301.1, YP_148605.1, YP_004340432.1, EFT09946.1, EFS80513.1, EFS51332.1, EFS42459.1, YP_003060895.1, YP_003059033.1, ZP_03305373.1, YP_847214.1, YP_004095847.1, YP_003338282.1, YP_003337256.1, YP_355846.1, YP_253131.1, ZP_08197563.1, ZP_08196283.1, ADW06447.1, YP_734091.1, NP_372233.1, NP_102173.1, ZP_08170259.1, EGD36706.1, EGD32748.1, ZP_08155540.1, YP_004142849.1, YP_002417649.1, YP_001301040.1, YP_002992892.1, YP_081348.1, YP_080482.1, YP_002476349.1, ZP_08115025.1, ZP_08114403.1, YP_003552869.1, YP_002358112.1, YP_575010.1, YP_477594.1, YP_474564.1, YP_130399.1, YP_129373.1, YP_123314.1, NP_810467.1, NP_646469.1, NP_626044.1, NP_391071.1 (kodiert durch SEQ ID NR: 11), ZP_08086822.1, ZP_08084776.1, ZP_08083119.1, ZP_08020768.1, ZP_08013590.1, ZP_08011832.1, YP_003783744.1, YP_002781576.1, YP_002780533.1, ZP_02195873.1, NP_797482.1, ZP_07645051.1, ZP_07643260.1, ZP_06611917.1, AAT40119.1, ZP_07864946.1, YP_004068409.1, YP_002796203.1, YP_002774420.1, YP_003600348.1, YP_003599946.1, YP_003565624.1, YP_003565223.1, YP_335198.1, YP_423850.1, YP_155059.1, ZP_07843538.1, ZP_07841226.1, ZP_06928932.1, ZP_05692073.1, ZP_05687006.1, ZP_04867480.1, YP_775531.1, CBE70214.1, ZP_07721182.1, ZP_04302850.1, ZP_04298961.1, ZP_04287684.1, ZP_04277177.1, ZP_04248389.1, ZP_04235899.1, ZP_02159718.1, ZP_02152178.1, YP_003974610.1, YP_003546595.1, YP_002317127.1, ZP_07313778.1, ZP_07302778.1, ZP_07298850.1, CBK69442.1, YP_003413835.1, YP_003595089.1, ZP_06807811.1, YP_003582455.1, YP_003464731.1, YP_003496397.1, YP_003421918.1, CBL07274.1, CBK64956.1, YP_003508515.1, AAL87460.1, AAC23579.1, AAC23578.1, AAC23577.1, ACU78652.1, YP_003471439.1, YP_003452777.1, ZP_06384971.1, ACY25368.1, ABC26869.1, AAP44334.1, EEZ80018.1, ZP_05110458.1, 1PJB_A, ZP_04717201.1, ZP_04689103.1, CAO90307.1, CAM75354.1, CAA44791.1, BAA77513.1, EGR96638.1, EGL90046.1, YP_004510847.1, ZP_08450330.1, YP_003387804.1, YP_003058152.1, EFS74272.1, EFS67128.1, ZP_06844564.1, YP_826658.1, YP_001195249.1, YP_003095978.1, YP_469292.1, YP_004442054.1, YP_004461174.1, YP_004055616.1, YP_003576656.1, YP_003094537.1, YP_001295973.1, AEE71143.1, YP_004447480.1, YP_003761844.1, YP_040853.1, YP_003154888.1, YP_003142045.1, YP_002280953.1, NP_371963.1, NP_422368.1, EGC98966.1, EGC76398.1, YP_004263661.1, YP_004252039.1, YP_679036.1, YP_499973.1, ZP_08054972.1, ZP_08053009.1, ZP_04067276.1, ZP_03968868.1, ZP_03963857.1, ZP_03933079.1, ZP_03497046.1, ZP_06668924.1, ZP_06667106.1, ZP_06324464.1, ZP_06196777.1, ZP_05114159.1, ZP_05083968.1, ZP_05070370.1, ZP_05030022.1, ZP_04673064.1, ZP_03517011.1, ZP_03505783.1, XP_001310698.1, ABK27691.1 und CAB59281.2 und Varianten davon umfassen. Für die von der Alanindehydrogenase katalysierte Reaktion ist die Anwesenheit nicht nur von Pyruvat, das als Teil des Primärstoffwechsels von jeder als Ganzzellkatalysator in Frage kommenden Zelle gebildet wird, erforderlich, sondern auch von Ammonium. Letzteres wird typischerweise in Form von anorganischen Stickstoffsalzen, beispielsweise Ammoniumsalzen, Nitraten o. ä. bereitgestellt. Bevorzugt wird dem wässrigen Reaktionsmedium ein Ammoniumsalz zugesetzt, z.B. Ammoniumchlorid.

Weiterhin ist es vorteilhaft, wenn der erfindungsgemäße Ganzzellkatalysator eine Alkanhydroxylase und optional weitere, für die Aktivität der Alkanhydroxylase essentielle Enzyme exprimiert, insbesondere für den Fall, dass als Substrate zur Herstellung eines Diamins eine Fettsäure mit nur einer endständigen Carboxyfunktion eingesetzt wird. Die Alkanhydroxylase und/oder eine zusätzlich exprimierte Alkoholdehydrogenase oxidieren das endständige Kohlenstoffatom dann bis zur Aldehydgruppe, die anschließend von der Transaminase aminiert werden kann, oder bis zur Carboxygruppe, die durch eine α-Dioxygenase oder die Kombination aus Fettsäurereduktase und einer Phosphopantheteinyl-Transferase in eine endständige Aldehydgruppe umgewandelt wird, welche anschließend von der Transaminase aminiert werden kann. In einer bevorzugten Ausführungsform wird unter dem Begriff "Alkanhydroxylase", wie hierin verwendet, ein Enzym verstanden, das die Hydroxylierung von unsubstituierten linearen Alkylresten umfassend wenigstens sechs, bevorzugt zwölf Kohlenstoffstoffreste katalysiert.

Als Alkanhydroxylasen sind erfindungsgemäß zahlreiche Oxidationssysteme geeignet, wie sie u. a. in der PCT/EP 2008/067447 beschrieben sind. In einer bevorzugten Ausführungsform handelt es sich bei der Alkanhydroxylase um eine Cytochrom P450-Monooxygenase der CYP153-Familie. In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. Die genannten Datenbankcodes beziehen sich dabei, wie durchgehend in dieser Anmeldung, auf die NCBI (National Center for Biotechnology Information, Bethesda, USA)-Datenbanken, genauer gesagt die am 21. November 2012 online verfügbare Version. In einer bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante davon, die bevorzugt Alkanhydroxylaseaktivität aufweist.

Zur optimalen Versorgung der Cytochrom P450-Monooxygenase der CYP153-Familie mit Elektronen aus dem Reduktionsmittel, bevorzugt NADH, ist es bevorzugt, dass die Zelle die Alkanhydroxylase zusammen mit funktionell mit ihr wechselwirkender Ferredoxin-Reduktase und funktionell mit ihr wechselwirkendem Ferredoxin exprimiert wird. Dabei kann es sich um isolierte oder bei der Verwendung eines Ganzzellkatalysators um co-exprimierte Polypeptide oder um N- oder C-terminal mit der Cytochrom P450-Monooxygenase der CYP153-Familie fusionierte Polypeptide handeln. Ob eine Ferredoxin-Reduktase oder ein Ferredoxin mit einer gegebenen Cytochrom P450-Monooxygenase der CYP153-Familie mit einander funktionell wechselwirken, kann der Fachmann leicht dadurch feststellen, ob das Reduktionsmittel in Gegenwart eines Alkansubstrates und der drei Polypeptide effizienter als für den Fall, dass wenigstens eines der drei fehlt, oxidiert wird. Alternativ kann der von Scheps, D., Malca, H., Hoffmann, B., Nestl, B. M, und Hauer, B. (2011) Org. Biomol. Chem., 9, 6727 beschriebene Enzymtest verwendet werden, der im Fall funktionell wechselwirkender Polypeptide eine deutliche Erhöhung der Reaktionsgeschwindigkeit zeigt. In einer besonders bevorzugten Ausführungsform stammen die Cytochrom P450-Monooxygenase der CYP153-Familie, das Ferredoxin und die Ferredoxin-Reduktase aus dem gleichen Organismus. In einer besonders bevorzugten Ausführungsform handelt es sich um die Ferredoxin-Reduktase aus *Alcanivorax borkumensis* SK2 (Datenbankcode YP_691923) oder eine Variante davon, das Ferredoxin aus *Alcanivorax borkumensis* SK2 (Datenbankcode YP_691920) oder eine Variante davon und die Cytochrom P450-Monooxygenase der CYP153-Familie aus *Alcanivorax borkumensis* SK2 (Datenbankcode YP_691921) oder eine Variante davon.

In einer weiteren bevorzugten Ausführungsform handelt es sich bei der Alkanhydroxylase um eine AlkB-Monooxygenase. AlkB stellt eine zunächst aus dem AlkBGT-System aus *Pseudomonas putida* Gpo1 bekannt gewordene Oxidoreduktase dar, die von zwei weiteren Polypeptiden, AlkG und AlkT, abhängig ist. AlkT wird als FAD-abhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. In einer bevorzugten Ausführungsform wird unter dem Begriff "AlkB-Monooxygenase" ein Polypeptid mit einer Sequenzhomologie von wenigstens, in der Reihenfolge zunehmender Bevorzugung angegeben, 75, 80, 85, 90, 92, 94, 96, 98 oder 99 % zur Sequenz des AlkB von *Pseudomonas putida* Gpo1 (Datenbankcode: CAB54050.1; dieser Datenbankcode stammt wie alle weiteren in der Anmeldung verwendeten aus dem Stand der Technik, nämlich aus der NCBI Datenbank, genauer dem am 15. Oktober 2012 online verfügbaren Release) mit der Fähigkeit, Alkane zu oxidieren. In einer besonders bevorzugten Ausführungsform handelt es sich bei der AlkB-Monooxygenase um eine mit den AlkG (CAB54052.1)- und AlkT (CAB54063.1)-Polypeptiden aus *Pseudomonas putida* Gpo1 funktionell zusammenwirkende, Alkane oxidierende Oxidoreduktase. Zur optimalen Versorgung der AlkB-Alkanhydroxylase mit Elektronen ist es bevorzugt, dass die Zelle die Alkanhydroxylase zusammen mit funktionell mit ihr wechselwirkenden Hilfsproteinen, bevorzugt AlkG und/oder AlkT oder jeweils Varianten davon exprimiert wird, wobei es sich in einer besonders bevorzugten Ausführungsform wiederum um AlkG (CAB54052.1)- und AlkT (CAB54063.1)-Polypeptide aus *Pseudomonas putida* Gpo1 handelt.

Bei der Verwendung eines Ganzzellkatalysators kann sich das Problem stellen, dass ein Substrat mit einem intrazellulär lokalisierten Enzym in Kontakt gebracht werden muss, damit es zur erwünschten Reaktion kommt. Im Falle langkettiger Alkane und Derivate davon ist bevorzugt, dass der Ganzzellkatalysator ein Polypeptid der AlkL-Familie aufweist. AlkL ist ein Membranprotein aus *Pseudomonas putida,* das langkettige Fettsäuren und Derivate davon in bakterielle Zellen importieren kann. In einer bevorzugten Ausführungsform handelt es sich bei einem "Polypeptid der AlkL-Familie", wie hierin verwendet, um ein Polypeptid, das über eine Länge von 230 aufeinander abfolgenden Aminosäuren eine wenigstens 80, bevorzugt 90, noch bevorzugter 90%ige Sequenzidentität zu AlkL aus *Pseudomonas putida* (Datenbankcode CAB69081) oder eine Variante von AlkL aus *Pseudomonas putida* und bevorzugt die Fähigkeit aufweist, den Import von langkettigen Alkanen ins Innere einer Zelle zu unterstützen. In einer weiteren Ausführungsform handelt es sich bei einem "Polypeptid der AlkL-Familie", wie hierin verwendet, um eine in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und bevorzugt zusätzlich AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobacter manganoxydans* Mnl7-9 (Datenbankcode ZP_09158756), *Caulobacter* sp. K31 (Datenbankcode YP_001672217), *Pseudomonas oleovorans* (Datenbankcode Q00595) und Varianten davon.

Die Lehre der vorliegenden Erfindung kann nicht nur unter Verwendung von Makromolekülen mit der exakten Aminosäure- oder Nukleinsäuresequenz, auf die hierin Bezug genommen wird, bzw. nicht nur unter Verwendung von einer Zelle mit relativ zum jeweiligen Wildtyp verringerter Aktivität eines Polypeptides mit der exakten Aminosäuresequenz, auf die hierin Bezug genommen wird, ausgeführt werden, sondern auch unter Verwendung einer Variante derartiger Makromoleküle oder von einer Zelle mit einer relativ zum jeweiligen Wildtyp der jeweiligen Zelle verringerten Aktivität einer Variante des Polypeptids, die durch Deletion, Addition oder Substitution einer oder mehr als einer Aminosäuren oder Nukleinsäuren erhalten werden kann. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die eine Sequenz umfasst oder darstellt, welche mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder solche lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3rd edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatische Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}-Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Protease. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in F M Ausubel (1995), Current Protocols in Molecular Biology. John Wiley & Sons, Inc. beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

In einer bevorzugten Ausführungsform weist die erfindungsgemäß verwendete Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase, einen Fettsäureimporter oder Varianten davon umfasst. Die β-Oxidation von Fettsäuren ist ein weit verbreiteter Stoffwechselweg, der es prokaryontischen und eukaryontischen Organismen gleichermaßen erlaubt, Fettsäuren zu oxidieren und die darin enthaltene chemische Energie dem Stoffwechsel verfügbar zu machen. Im weiteren Sinn beginnt sie mit der Aufnahme einer Fettsäure in die Zelle. Dort wird die Fettsäure, sofern die Bedingungen es erfordern, zunächst an der β-Position des CoA-Fettsäureesters durch eine Acyl-CoA-Dehydrogenase, im Falle von *E. coli* FadE, oxidiert. Ein ähnliches Molekül kann alternativ auch aus einer doppelt ungesättigten Fettsäure durch Reduktion mittels einer 2,4-dienoyl-CoA-Reduktase, bei *E. coli* FadH, gebildet werden. Ein multifunktionelles Enzym, die Enoyl-CoA-Hydratase/3-Hydroxyacyl-CoA-Dehydrogenase, bei *E. coli* FadB, katalysiert anschließend die Hydratisierung unter Bildung des sekundären Alkohols und dessen anschließende Oxidation zum Keton. Im letzten Schritt katalysiert eine 3-Ketoacyl-CoA-Thiolase, im Falle von *E. coli* FadA, die Spaltung des Ketoacyl-CoA mit dem Ergebnis, dass Acetyl-CoA und ein im Vergleich zum Ausgangsmolekül um zwei Kohlenstoffatome verkürzter CoA-Ester der Fettsäure freigesetzt werden. Sofern es sich nicht ebenfalls um Acetyl-CoA handelt, kann letzterer erneut in den β-Oxidationszyklus eingespeist und unter Oxidation verkürzt werden. An der Regulation der β-Oxidation von Fettsäuren ist auch FadR beteiligt, ein Regulator des Fad-Operons, der die für den Abbau von Fettsäuren erforderlichen Gene umfasst, ohne dass FadR eine Reaktion der β-Oxidation katalysieren würde. In einer bevorzugten Ausführungsform wird unter dem Begriff "Enzym, das eine der Reaktionen der β-Oxidation von Fettsäuren katalysiert" ein jegliches Enzym verstanden, das direkt mit dem Fettsäuresubstrat oder einem auf dem Weg zum Acetyl-CoA daraus entstehenden Molekül wechselwirkt, bevorzugt es als Substrat erkennt, und seine Umwandlung zu einem auf diesem Abbauweg näher am Acetyl-CoA liegenden Stoffwechselprodukt katalysiert, bevorzugt einschließlich des Fettsäureimporters, der die Aufnahme der Fettsäure in die Zelle bewerkstelligt. Beispielsweise zählt zu diesen Enzymen nach der vorangegangenen Definition die Acyl-CoA-Dehydrogenase, da sie mit dem Fettsäure-CoA-Ester wechselwirkt und dessen Umwandlung zum Enyol-CoA katalysiert, das auf dem Stoffwechselweg der β-Oxidation näher am Acetyl-CoA liegt als der Fettsäure-CoA-Ester. In einer besonders bevorzugten Ausführungsform wird unter dem Begriff "Enzym, das eine der Reaktionen der β-Oxidation von Fettsäuren katalysiert", wie hierin verwendet, jedes Enzym aus der Gruppe verstanden, die die Genprodukte FadA, FadB, FadD, FadL und FadE aus *E. coli* und/oder deren Varianten oder Homologa aus anderen Organismen umfasst. Die Genprodukte FadA, FadB, FadD, FadL und FadE aus *E. coli* ebenso wie Varianten und Homologa aus zahlreichen weiteren biotechnologisch nutzbaren Organismen und ihre Nukleinsäure- und Polypeptidssequenzen sind im Stand der Technik beschrieben, beispielsweise FadA unter Zugangsnummer AP009048.1, FadB unter Zugangsnummer BAE77457.1, FadD unter Zugangsnummer BAA15609.1, und FadE unter Zugangsnummer BAA77891.2. Der Stand der Technik offenbart zahlreiche Tests, die speziell für die Messung der Aktivität von Enzymen geeignet sind, die eine der Reaktionen der β-Oxidation von Fettsäuren katalysieren, beispielsweise in K Kameda & W D Nunn (1981) J. Biol. Chem. 256, 5702-5707, Hi Marrakchi, W E DeWolf, C Quinn, J West, B J Polizzi, C Y So et al. (2003) Biochem. J. 370, 1055-1062, Lobo et al. (2001) und X Yu, T Liu, F Zhu, and C Khosla (2011) PNAS, *elektronische Veröffentlichung vor Drucklegung.*

Für die Effektivität des erfindungsgemäßen Ganzzellkatalysators ist es von Vorteil, wenn das umzusetzende Substrat, bevorzugt die Carbonsäure oder Dicarbonsäure oder ein Monoester davon, leicht mit den erfindungsgemäß erforderlichen Enzymen, die im Inneren des Ganzzellkatalysators lokalisiert sind, in Kontakt treten kann. Entscheidend ist daher, dass das Substrat ins Innere der Zelle gelangen kann. Um dies zu erleichtern, ist bevorzugt, dass der Ganzzellkatalysator einen Fettsäureimporter, im Falle eines bakteriellen, insbesondere Gram-negativen Ganzzellkatalysators, besonders bevorzugt den Fettsäureimporter FadL (Datenbankcode: BAA16205.1) oder eine Variante exprimiert, bevorzugt in einer Konzentration und mit einer Aktivität, die gegenüber der Aktivität des Wildtyps des entsprechenden Ganzzellkatalysators erhöht ist. Der Erhöhung der Aktivität eines Polypeptids gegenüber dem Wildtyp der Zelle kann über verschiedene, dem Fachmann routinemäßig zugängliche Wege erreicht werden, beispielsweise den Einbau zusätzlicher, funktionell mit einem Promotor verbundener Kopien der für das Polypeptid kodierenden Nukleotidsequenz oder den Austausch des natürlichen gegen einen stärkeren Promotor.

Es hat sich gezeigt, dass die Amine und Diamine erfindungsgemäß in höherer Ausbeute und Reinheit produziert werden, wenn der Hintergrund an im Ganzzellkatalysator endogen exprimierten Enzymen derart optimiert ist, dass die Aktivität von endogenen Enzymen verringert oder ausgeschaltet ist, die Edukte, Zwischenprodukte oder Produkte des erfindungsgemäßen Verfahrens oder unter Verwendung der erfindungsgemäßen Zelle, bevorzugt Methylester von ω-Aminocarbonsäuren, ω-Hydroxycarbonsäuren, ω-Oxocarbonsäuren und Dicarbonsäuren, auf Stoffwechselwegen abbauen oder anderweitig modifizieren, die von der Entstehung des erwünschten Produktes wegführen. Vor diesem Hintergrund kann es vorteilhaft sein, wenn es sich bei dem erfindungsgemäßen Ganzzellkatalysator um eine Zelle handelt, die ihrem Wildtyp gegenüber eine verringerte Aktivität der Esterase BioH [Datenbankcode YP_492020.1] oder einer Variante davon aufweist. Derartige Zellen mit verringerter BioH-Aktivität, ihre Herstellung und Tests zur Aktivitätsbestimmung sind in der Europäischen Patentanmeldung EP 12007663.3 beschrieben.

Für den Fall, dass als Carbonsäure, Dicarbonsäure oder Monoester davon Mischungen von Verbindungen eingesetzt werden, bei denen die endständigen Carboxygruppen in hohem Maße, bevorzugt zu wenigstens 50, 60, 70, 80, 90, 95 oder 99 Prozent in Form eines Esters vorliegt, beispielsweise aus Gründen der besseren Verfügbarkeit dieser Substrate oder der Toxizität freier Carbonsäuren oder Dicarbonsäuren, kann der Monoester durch teilweise oder vollständige Hydrolyse von vollständig veresterten Dicarbonsäuren bzw. die freie Carbonsäure durch teilweise oder vollständige Hydrolyse von vollständig veresterten Carbonsäuren bereitgestellt werden. Für diesen Fall es vorteilhaft sein, die Kapazität der Zelle zur Esterhydrolyse durch Überexpression einer geeigneten Esterase zu steigern. In einer bevorzugten Ausführungsform wird dazu die Aktivität der Esterhydrolase BioH oder einer Variante davon gegenüber dem Wildtyp des eingesetzten Ganzzellkatalysators erhöht, besonders bevorzugt durch Überexpression. Der entsprechende Monoester und/oder die unveresterte Carbonsäure oder Dicarbonsäure wird dann *in situ* durch Esterhydrolyse bereitgestellt. Die teilweise oder vollständige Hydrolyse einer vollständig veresterten Dicarbonsäure kann auch durch chemisch katalysierte Hydrolyse erfolgen, beispielsweise bei niedrigen pH-Werten.

Der erfindungsgemäße Ganzzellkatalysator kann in einem Verfahren zur Umsetzung von einer Carbonsäure oder Dicarbonsäure oder eines Monoesters davon zum entsprechenden Amin oder Diamin, bevorzugt eingesetzt werden, wobei es sich bei der Carbonsäure oder Dicarbonsäure oder dem Monoester davon um eine Verbindung der Formel (I)

R¹-A-COOR² (I)

handelt, wobei R¹ aus der Gruppe ausgewählt ist, die -H und COOR³ umfasst, wobei R² und R³ jeweils und unabhängig voneinander aus der Gruppe ausgewählt sind, die H, Methyl, Ethyl und Propyl umfasst, mit der Maßgabe, dass wenigstens einer der Reste R² und R³ H ist, wobei A eine unverzweigte, verzweigte, lineare, zyklische, substituierte oder unsubstituierte Kohlenwasserstoffgruppe mit wenigstens vier Kohlenstoffatomen darstellt. In einer bevorzugten Ausführungsform handelt es sich bei A um eine Struktur der Formel -(CH₂)ₙ-, wobei n bevorzugt 4, 5, 6, 7, 8, 9, 10, 11, 12, 13, 14, 15, 16, 17, 18, 19, 20, 21, 22, 23, 24, 25, 26, 27, 28, 29 oder 30 ist. In einer bevorzugtesten Ausführungsform handelt es sich bei der Carbonsäure oder Dicarbonsäure um Laurinsäure oder ω-Carboxylaurinsäure. In einer weiteren bevorzugtesten Ausführungsform handelt es sich bei der Carbonsäure um eine Carbonsäure der Formel CH₃-(CH₂)ₙ-COOH, wobei n bevorzugt 4, 5, 6, 7, 8, 9, 10, 11, 12, 13, 14, 15, 16, 17, 18, 19, 20, 21, 22, 23, 24, 25, 26, 27, 28, 29 oder 30 ist, bevorzugt um Hexansäure oder Decansäure. In einer weiteren bevorzugtesten Ausführungsform handelt es sich bei der Carbonsäure oder Dicarbonsäure um eine Dicarbonsäure der der Formel HOOC-(CH₂)ₙ-COOH, wobei n bevorzugt 4, 5, 6, 7, 8, 9, 10, 11, 12, 13, 14, 15, 16, 17, 18, 19, 20, 21, 22, 23, 24, 25, 26, 27, 28, 29 oder 30 ist, bevorzugt um ω-Carboxyhexansäure oder ω-Carboxydecansäure. In einer weiteren bevorzugtesten Ausführungsform handelt es sich bei der Carbonsäure oder Dicarbonsäure um ω-Carboxytetradecansäure. Entsprechend handelt es sich bei dem erfindungsgemäß hergestellten Amin bzw. Diamin bevorzugt um eine Verbindung der Formel CH₃-(CH₂)ₙ-NH₂ bzw. NH₂-(CH₂)ₙ-NH₂, wobei n jeweils bevorzugt 4, 5, 6, 7, 8, 9, 10, 11, 12, 13, 14, 15, 16, 17, 18, 19, 20, 21, 22, 23, 24, 25, 26, 27, 28, 29 oder 30 ist.

Mit Hinblick auf die Carbonsäure oder Dicarbonsäure oder den Monoester davon wie auf jede in dieser Anmeldung beschriebene chemische Verbindung gilt, dass die jeweilige angegebene Formel sämtliche Salze, protonierte oder deprotonierte der jeweiligen Verbindung umfasst. Beispielsweise umfasst die Laurinsäure nicht nur die protonierte Form, sondern auch das Salz Laureat mit sämtlichen Kationen, beispielsweise Natriumlaureat.

Das erfindungsgemäße Verfahren erfordert, dass die für das erfindungsgemäße Verfahren eingesetzten Enzyme, optional in Form des erfindungsgemäßen Ganzzellkatalysators bereitgestellt, mit der Carbonsäure oder Dicarbonsäure oder dem Monoester davon in einer wässrigen Lösung kontaktiert werden. In einer bevorzugten Ausführungsform wird unter dem Begriff "Kontaktieren", wie hierin verwendet, verstanden, dass das jeweilige Enzyme in unmittelbaren Kontakt mit seinem Substrat gelangt, insbesondere ohne dass physikalische Barrieren wie impermeable Membranen oder dergleichen zwischengeschaltet sind. Das Kontaktieren geschieht im einfachsten Fall dadurch, dass das Substrat zu einer wässrigen Lösung gegeben wird, in der sich das Enzym oder der Ganzzellkatalysator befindet.

Zur Ausführung der erfindungsgemäßen Lehre eignet sich eine Reaktionsmischung umfassend den erfindungsgemäßen Ganzzellkatalysator 8 in wässriger Lösung sowie eine Carbonsäure oder Dicarbonsäure oder einen Monoester davon der Formel (I), wobei R¹ aus der Gruppe ausgewählt ist, die -H COOR³ umfasst, wobei R² und R³ jeweils und unabhängig voneinander aus der Gruppe ausgewählt sind, die H, Methyl, Ethyl und Propyl umfasst, mit der Maßgabe, dass wenigstens einer der Reste R² und R³ H ist, wobei A eine unverzweigte, verzweigte, lineare, zyklische, substituierte oder unsubstituierte Kohlenwasserstoffgruppe mit wenigstens vier Kohlenstoff darstellt, bevorzugt die Formel - (CH₂)ₙ -, wobei n wenigstens 4, besonders bevorzugt wenigstens 10 ist. Die wässrige Lösung muss, beispielsweise mit Hinblick auf Zusammensetzung, pH-Wert und Temperatur, dabei derart beschaffen sein, dass sie die Lebensfähigkeit oder zumindest die katalytische Fähigkeit des Ganzzellkatalysators wenigstens zeitweilig unterstützt. Dem Fachmann sind zahlreiche als wässrige Lösung geeignete wässrige Kulturmedien bekannt, die für die Erhaltung oder Kultivierung von Zellen, insbesondere biotechnologisch bedeutsamer Zellen, geeignet sind. Darunter fallen gleichermaßen Vollmedien wie LB-Medien, Minimalmedien wie M9-Medien sowie Selektivmedien, beispielsweise solche, die eine hohe Salzkonzentration enthalten und daher nur das Wachstum halophiler oder zumindest halotoleranter Organismen ermöglichen. In einer bevorzugten Ausführungsform wird unter dem Begriff "wässriges Kulturmedium", wie hierin verwendet, ein Reaktionsmedium auf Wasserbasis verstanden, das mit Hinblick auf sämtliche relevante Faktoren, insbesondere pH-Wert, Salzgehalt und Temperatur, derart beschaffen ist, das es die Lebensfähigkeit darin enthaltener Zellen, bevorzugt Mikroorganismen, erhält oder fördert und sowohl wässriges Kulturmedium als auch hydrophobe organische Phase in flüssiger Form vorliegen. Die Temperaturansprüche verschiedener biotechnologisch bedeutsamer Zellen können mikrobiologischen und molekularbiologischen Lehrbüchern entnommen werden, z.B. Fuchs/Schlegl, 2008. In einer bevorzugten Ausführungsform liegt der pH-Wert des wässrigen Kulturmediums zum Zeitpunkt des Kontaktierens zwischen 4 bis 9, bevorzugter zwischen 4,5 bis 8,5, am bevorzugtesten zwischen 6,5 und 7,5. In einer weiteren bevorzugten Ausführungsform liegt die Temperatur zwischen 0 und 45 °C, bevorzugter zwischen 15 und 40 °C, am bevorzugtesten zwischen 20 und 37 °C. Die Reaktionsmischung ist typischerweise in einem Fermenter enthalten. Als Fermenter kann ein jedes Reaktionsgefäß fungieren, das sterilisiert, bevorzugt autoklaviert werden kann und die Anzucht des Ganzzellkatalysators, Belüftung und Steuerung der Reaktionsbedingungen, beispielsweise des Sauerstoffgehalts und der Temperatur, erlaubt.

In einer bevorzugten Ausführungsform umfasst die Reaktionsmischung zusätzlich zur wässrigen Lösung eine hydrophobe organische Phase. Diese kann ein organisches Lösungsmittel und/oder einen hydrophoben flüssigen Kationenaustauscher zur Entfernung der ω-Aminofettsäure aus der wässrigen Lösung umfassen. Geeignete Lösungsmittel und Kationenaustauscher sind in der EP11191520.3 beschrieben.

Die vorliegende Erfindung wird weiterhin durch die folgenden Figuren und nicht beschränkenden Beispiele veranschaulicht, denen weitere Merkmale, Ausführungsformen, Aspekte und Vorteile der vorliegenden Erfindung entnommen werden können.

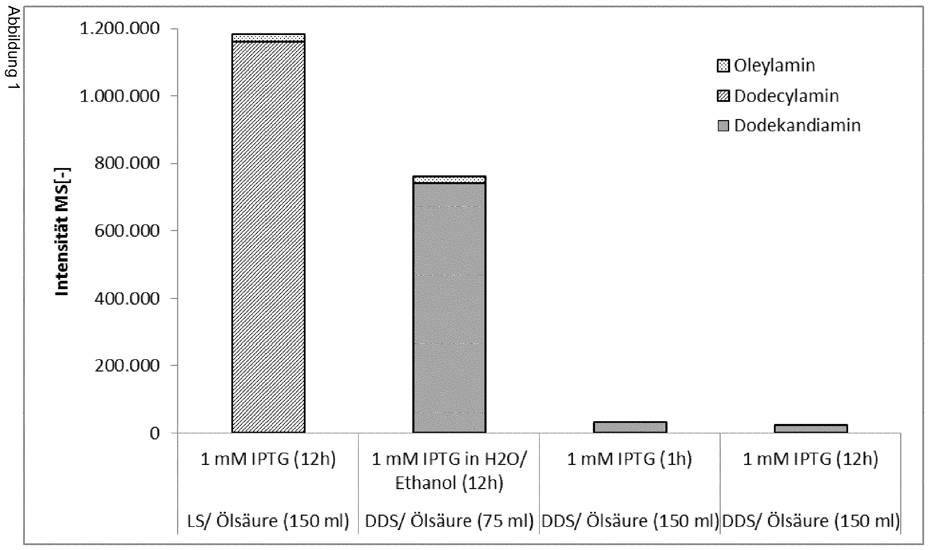

Abbildung 1: Nachweis von Mono- und Diaminen in der Fermentationsbrühe des Stammes *E.coli* W3110 pACYC{Placuv5}[carA_Ms-npt_Noc] / pJ281_alaDH_B.s._TA_C.v.(ct) nach 21,75h Prozesszeit.

### Beispiel 1

### Herstellung eines Expressionsvektors für die Expression der Gene MSMEG_2956 aus Mycobacterium smegmatis und npt aus Nocardia sp.

Zur Herstellung von Vektoren für die Coexpression von MSMEG_2956 (carA, SEQ ID Nr. 1), aus *Mycobacterium smegmatis* kodierend für eine Fettsäurereduktase (YP_887275.1) und mit *npt* (SEQ ID Nr. 2) aus *Nocardia sp.* kodierend für eine die Fettsäurereduktase phosphopantheteinylierenden Phosphopantheteinyl-Transferase (ABI83656.1) wurden beide Gene mittels PCR unter Einbringung homologer Bereiche zur Rekombinationsklonierung amplifiziert. Dabei diente genomische DNA des Spenderorganismus für die Amplifikation des Gens MSMEG_2956 und ein synthetisiertes DNA-Fragment für die Amplifikation des Gens *npt* als Vorlage. Die Gene liegen unter Kontrolle eines lacuv5-Promotors (SEQ ID Nr. 3), der ebenfalls mittels PCR ausgehend von einem vorhandenen Vektor unter Einbringung homologer Bereiche für die Rekombinationsklonierung amplifiziert wurde.

Dabei kamen folgende Oligonukleotide zum Einsatz:
Plac_H1_fw: 5'-TTATGCGACTCCTGCTGGCTATGGTGGGATTTCC-3' (SEQ ID Nr. 4)
Plac_H2_rv: 5'-GATCGTCATATGCCACTCTCCTTGGTTCC-3' (SEQ ID Nr. 5)
carA_H2_fw: 5'-TGGCATATGACGATCGAAACGCGCG-3' (SEQ ID Nr. 6)
carA_H3_rv: 5'-TCCTTCTCTTACAGCAATCCGAGCATCT-3' (SEQ ID Nr. 7)
npt_H3_fw: 5'-GCTGTAAGAGAAGGAGTTCTATCATGATCGAG-3' (SEQ ID Nr. 8)
npt_H4_rv: 5'-GCAGCCTAGGTTAATTTATCAGGCGTACGCGATCG-3' (SEQ ID Nr. 9)

Folgende Parameter wurden für die PCR zur Amplifikation des P_{lacuv5} und des Gens *npt* eingesetzt: 1 x: initiale Denaturierung, 98°C, 0:30 min; 35 x: Denaturierung, 98°C, 0:10 min, Annealing, 55°C, 0:20 min; Elongation, 72°C, 0:15 min; 1 x: terminale Elongation, 72 °C, 10 min. Für die Amplifikation des Gens MSMEG_2956 wurden folgende Parameter eingesetzt: 1 x: initiale Denaturierung, 98°C, 0:30 min; 35 x: Denaturierung, 98°C, 0:10 min, Annealing, 65°C, 0:20 min; Elongation, 72°C, 1 min; 1 x: terminale Elongation, 72 °C, 10 min. Für die Amplifikation wurde der Phusion™ High-Fidelity Master Mix von New England Biolabs (Frankfurt) entsprechend den Empfehlungen des Herstellers verwendet. Jeweils 50 µl der PCR-Reaktionen wurden anschließend auf einem 1%igen TAE-Agarosegel aufgetrennt. Die Durchführung der PCR, der Agarose-Gel-Elektrophorese, Ethidiumbromidfärbung der DNA und Bestimmung der PCR-Fragmentgrößen erfolgte in dem Fachmann bekannter Art und Weise. In allen Fällen konnten PCR-Fragmente der erwarteten Größe amplifiziert werden. Diese betrugen P_{lacuv5} 325 Basenpaare, MSMEG_2956 3,5 Kilobasenpaare und *npt* 718 Basenpaare. Zur Isolierung der DNA aus dem Agarosegel wurde die Ziel-DNA mit einem Skalpell aus dem Gel herausgeschnitten und mit dem *QiaQuick Gel extraction Kit* nach Anleitung des Herstellers (Qiagen, Hilden) aufgereinigt. Die aufgereinigten PCR-Produkte wurden in einen *Eco*NI- und *Pac*I-geschnittenen Vektor pACYCDuet-1 (Merck, Darmstadt) mittels Rekombination unter Verwendung des Geneart® Seamless Cloning and Assembly Kit nach Anleitung des Herstellers (Life Technologies, Carlsbad, CA, USA) kloniert. Die Transformation chemisch kompetenter *E.coli* DH10β (New England Biolabs, Frankfurt) erfolgte nach dem Fachmann bekannter Art und Weise. Die korrekte Insertion der Zielgene wurde durch Restriktionsanalyse überprüft und die Authentizität der eingebrachten Genedurch DNA-Sequenzierung bestätigt. Der fertiggestellte Expressionsvektor wurde als pACYC{Placuv5}[carA_Ms-npt_Noc] (SEQ ID Nr. 10) bezeichnet.

### Beispiel 2

### Herstellung eines Expressionsvektors für die Co-Expression der Gene ald aus Bacillus subtilis und Cv2025 aus Chromobacterium violaceum

Zur Herstellung eines *E. coli*-Expressionsvektors für die Gene *ald* (SEQ ID Nr. 11) aus *Bacillus subtilis* kodierend für eine Alanindehydrogenase (NP_391071.1) und *Cv_2025* (SEQ ID Nr. 12) aus *Chromobacterium violaceum* kodierend für eine Transaminase (NP_901695.1) wurde das Gen ald aus *Bacillus subtilis* im Austausch gegen das Gen *ald* aus *Bacillus sphaericus* in den *E.coli*-Expressionsvektor pJ281_alaD_Bsp_TA_C.v.(ct) (Sequenz und Herstellung vergleiche Beispiel 1 der WO/2013/024114 und die dort aufgeführte SEQ ID Nr. 17) kloniert. Das Gen *ald aus Bacillus subtilis* wurde per PCR aus chromosomaler DNA des Stammes *Bacillus subtilis* str. 168 amplifiziert. Dabei kamen folgende Oligonukleotide zum Einsatz:
- alaDH_pCR22_fw:: 5'-ATGATCATAGGGGTTCCTAAAGAG-3'
(SEQ ID Nr. 13)
- alaDH_pCR22_rev:: 5'-TTAAGCACCCGCCACAGATG-3'
(SEQ ID Nr. 14)

Folgende Parameter wurden für die PCR eingesetzt: 1x: initiale Denaturierung, 98 °C, 0:30 min; 35 x: Denaturierung, 98 °C, 0:10 min, Annealing, 65°C, 0:30 min; Elongation, 72 °C, 0:20 min; 1 x: terminale Elongation, 72 °C, 10 min. Für die Amplifikation wurde der Phusion™ High-Fidelity Master Mix von New England Biolabs (Frankfurt) entsprechend den Empfehlungen des Herstellers verwendet. Jeweils 50 µl der PCR-Reaktionen wurden anschließend auf einem 1%igen TAE-Agarosegel aufgetrennt. Die Durchführung der PCR, der Agarose-Gelelektrophorese, Ethidiumbromidfärbung der DNA und Bestimmung der PCR-Fragmentgrößen erfolgte in dem Fachmann bekannter Art und Weise. Das PCR-Fragment zeigte die erwartete Größe von 1137 Basenpaaren und wurde mit dem Quick PCR Purification Kit von Qiagen (Hilden) nach Angaben des Herstellers aus dem PCR-Ansatz aufgereinigt. Für die Ligation des PCR Produktes mit dem Vektor wurden 5'-Phosphate mit Hilfe der Polynukleotidkinase (New England Biolabs, Frankfurt) an das PCR-Produkt angehängt. Dabei wurde die Empfehlung des Herstellers befolgt.

Der Vektor wurde mit den Restriktionsendonukleasen *Hin*dIII und *Nde*I verdaut, wodurch das enthaltene Gen *Bacillus sphaericus ald* entfernt wurde. Der Ansatz des Restriktionsverdaus wurde auf einem 1%igen TAE-Agarosegel aufgetrennt. Es konnten zwei Banden mit den Größen 5696 bp und 1124 bp identifiziert werden. Zur Isolierung der Vektor-DNA aus dem Agarosegel wurde die DNA-Bande von 5696 bp mit einem Skalpell aus dem Gel isoliert und mit dem Quick Gel Extraction Kit von Qiagen (Hilden) nach Angaben des Herstellers aufgereinigt. Für die Erzeugung von glatten Enden wurden die 5'-Überhänge der aufgereinigten Vektor-DNA mit Hilfe des Klenow-Fragmentes der DNA-Polymerase I (New England Biolabs, Frankfurt) aufgefüllt. Dabei wurden die Angaben des Herstellers befolgt. Das DNA-Fragment *Bacillus subtilis ald* mit 5'-Phosphatresten wurde in den Vektor mit glatten Enden ligiert. Der fertiggestellte E. coli-Expressionsvektor wurde als pJ281_alaDH_B.s._TA_C.v.(Ct) (SEQ ID Nr. 15) bezeichnet.

### Beispiel 3

### Herstellung eines E.coli-Stammes, der die Gene MSMEG_2956 aus Mycobacterium smegmatis und npt aus Nocardia sp., ald aus Bacillus subtilis und Cv2025 aus Chromobacterium violaceum überexprimiert

Zur Erzeugung eines *E.coli*-Stammes, welcher die Gene MSMEG_2956 aus *Mycobacterium smegmatis* kodierend für eine Fettsäurereduktase (YP_887275.1) und *npt* aus *Nocardia sp.* kodierend für eine die Fettsäurereduktase phosphopantheteinylierenden Phosphopantheteinyl-Transferase (ABI83656.1) in Kombination mit den Genen *ald* aus *Bacillus subtilis* kodierend für eine Alanindehydrogenase (NP_391071.1) und Cv2025 aus *Chromobacterium violaceum* kodierend für eine Transaminase (NP_901695.1) coexprimiert, wurde der Stamm *E.coli* W3110 mit den Plasmiden pACYC{Placuv5}[carA_Ms-npt_Noc] (SEQ ID Nr. 10) und pJ281_alaDH_B.s._TA_C.v.(ct) (SEQ ID Nr. 15) mittels Elektroporation transformiert und auf LB-Agar-Platten mit Chloramphenicol (50 µg/ml) und Kanamycin (50 µg/ml) ausplattiert. Transformanten wurden durch Plasmidpräparation und analytische Restriktionsanalyse bezüglich der Präsenz der korrekten Plasmide überprüft. Der erzeugte Stamm wurde als *E.coli* W3110 pACYC{Placuv5}[carA_Ms-npt_Noc] / pJ281_alaDH_B.s._TA_C.v.(ct) bezeichnet. Der Stamm wurde verwendet um seine Fähigkeit zur Produktion von Dodekandiamin ausgehend von Dodekandisäure und Dodecylamin ausgehend von Dodekansäure zu untersuchen. Das Genprodukt CarA, eine Fettsäurereduktase, die durch die überexprimierte Phosphopantetheinyltransferase *npt* aktiviert wird, setzt das Substrat Dodekansäure bzw. Dodekandisäure zum jeweiligen Aldehyd bzw. Dialdehyd um. Die Funktion des Genproduktes Cv_2505 besteht darin, das (Di-)aldehyd endständig zum Dodecylamin bzw. Dodekandiamin umzusetzten. Der für die Aminierungsreaktion benötigte Aminodonor Alanin wird von dem Genprodukt *ald* aus Pyruvat zur Verfügung gestellt.

### Beispiel 4

### Produktion von Dodekandiamin und Dodecylamin durch E.coli Stämme mit einem Expressionsvektor für die Gene MSMEG_2956 aus Mycobacterium smegmatis und npt aus Nocardia sp. in Kombination mit einem Expressionsvektor für die Gene ald aus Bacillus subtilis und Cv_2025 aus Chromobacterium violaceum

Der in Beispiel 3 erzeugte Stamm wurde verwendet, um seine Fähigkeit zur Produktion von Dodecylamin und Dodekandiamin zu untersuchen. Die Biotransformation von Dodekansäure bzw. Dodekandisäure zu Dodecylamin bzw. Dodekandiamin wurde im 8-fach Parallelfermentationssystem von DASGIP durchgeführt. Dabei wurde wie folgt vorgegangen: Für die Fermentation wurden 1L-Reaktoren verwendet. Die pH-Sonden wurden mittels einer Zwei-Punkt-Kalibration mit Maßlösungen von pH 4,0 und pH 7,0 kalibriert. Die Reaktoren wurden mit 300 mL Trinkwasser befüllt und 20 min bei 121°C autoklaviert, um Sterilität zu gewährleisten. Anschließend wurden die pO2-Sonden über Nacht (mindestens 6 h lang) am DASGIP-System polarisiert. Am nächsten Morgen wurde das Wasser unter der Clean Bench entnommen und durch 300 mL Hochzelldichtemedium mit 50 mg/L Chloramphenicol und 50 mg/L Kanamycin ersetzt. Im Folgenden wurden die pO2-Sonden mit einer Einpunkt-Kalibration (Rührer: 400 rpm / Begasung: 10 sL/h Luft) kalibriert und die Feed-, Korrekturmittel- und Induktionsmittelstrecken mittels Clean-in-Place gereinigt. Dazu wurden die Schläuche mit 70% Ethanol, anschließend mit 1 M NaOH, dann mit sterilem VE-Wasser gespült und zuletzt mit den jeweiligen Medien befüllt.

Der Dodekandiamin und Dodecylamin produzierende *E*. *coli*-Stamm wurden zuerst aus der Kryokultur in LB-Medium (25 mL in einem 100 mL Schikanekolben) mit den oben genannten Antibiotika über Nacht bei 37°C und 200 rpm ca. 18 h lang angezogen. Anschließend wurden 2 mL der Kultur in Hochzelldichtemedium (Glucose 15 g/L (30 mL / L einer separat autoklavierten 500 g/L Stammlösung mit 1% MgSO₄*7H₂O und 2,2% NH₄Cl), (NH₄)₂SO4 1,76 g/L, K₂HPO₄ 19,08 g/L, KH₂PO₄ 12,5 g/L, Hefeextrakt 6,66 g/L, Trinatriumcitrat-Dihydrat 2,24 g/L, Ammoniumeisencitrat-Lösung 17 mL/L einer separat autoklavierten 1% igen Stammlösung, Spurenelementlösung 5 mL/L separat autoklavierten Stammlösung (HCl (37 %) 36,50 g/L, MnCl₂*4H₂O 1,91 g/L, ZnSO₄*7H₂O 1,87 g/L, Ethylendiamintetraessigsäure-Dihydrat 0,84 g/L, H₃BO₃ 0,30 g/L. Na₂MoO₄*2H₂O 0,25 g/L, CaCl₂*2H₂O 4,70 g/L, FeSO₄*7H₂O 17,80 g/L, CuCl₂*2H₂O 0,15 g/L)) (25 mL in einem 100 mL Schikanekolben) mit den oben genannten Antibiotika überimpft und bei 37°C / 200 rpm weitere 5,5 h lang inkubiert.

Die Reaktoren wurden mit einer optischen Dichte von 0,1 angeimpft indem ein entsprechendes Volumen der Vorkultur in einer 5 mL Spritze (unter sterilen Bedingungen) aufgezogen wurde und die Reaktoren mittels Kanüle über ein mit 70% Ethanol überschichtetes Septum beimpft wurde.

Folgendes Standardprogramm wurde verwendet:

| DO-Regler | | pH-Regler | |
|---|---|---|---|
| Preset | 0% | Preset | 0 ml/h |
| P | 0,1 | P | 5 |
| Ti | 300 s | Ti | 200 s |
| Min | 0% | Min | 0 mlL/h |
| Max | 100% | Max | 40 mL/h |

| N (Rotation) | von | bis | XO2 (Gasmischung) | von | bis | F (Gasfluss) | von | bis |
|---|---|---|---|---|---|---|---|---|
| Wachstum und Biotransformation | 0% | 30% | Wachstum und Biotransformation | 0% | 100% | Wachstum und Biotransformation | 15% | 80% |
| | 400 rpm | 1500 rpm | | 21 % | 21% | | 6 sL/h | 72 sL/h |

| Script | |
|---|---|
| Trigger scharf | 31% DO (1/60h) |
| Induktion IPTG | 2 h nach Feedstart |
| Feedtrigger | 50% DO |
| Feedrate | 3 [mL/h] |

Das durchgeführte Experiment lässt sich in zwei Phasen gliedern, die Anzucht, bei der die Zellen eine bestimmte optische Dichte erreichen sollen, und die nachfolgende Biotransformation, in der nach Zugabe der Substrate Dodekansäure, Ölsäure bzw. Dodekandisäure eine Umsetzung zu Dodecylamin, Oleylamin bzw. Dodekandiamin von in der Expression gebildeten Enzyme stattfinden soll. Die pH-Werte wurden einseitig mit Ammoniak (12,5 %) auf pH 6,8 geregelt. Während Anzucht und Biotransformation wurde der gelöste Sauerstoff (DO, dissolved oxygen) in der Kultur über Rührerdrehzahl und Begasungsrate bei 30% geregelt. Die Fermentation wurde als Fed-Batch durchgeführt, wobei der Feedstart, 5 g/Lh Glucosefeed (500 g/L Glucose mit 1% MgSO₄*7H₂O und 2,2% NH₄Cl), über einen DO-Peak getriggert wurde. Mit Feedstart wurde auch die Temperatur von vorher 37°C auf 30°C gesenkt. Die Expression der Transaminase, Alanindehydrogenase, Carbonsäurereduktase und Phosphopantetheinyltranferase wurde 2 h nach Feedstart durch die automatische Zugabe von 1 mM IPTG induziert. Vor Start der Biotransformation wurde die optische Dichte der Kulturbrühen bestimmt.

Der Start der Biotransformationsphase erfolgte 1h bzw. 12 h nach Feedstart. Dazu wurden 150 mL oder 75 ml eines Gemisches aus Dodekansäure bzw. Dodekandisäure und Ölsäure (techn. 90%) als Batch zu der Fermentationsbrühe zugegeben. Um einen Aminogruppendonor für die Transaminase zur Verfügung zu stellen, wurde eine halbe Stunde vor Biotransformationsstart 5 mL einer 3M Ammoniumsulfatlösung zu der Fermentationsbrühe zugegeben. Zur Probennahme wurden 2 mL Fermentationsbrühe aus dem Kessel entnommen und ein Teil davon 1/20 in einem und in einem Gemisch aus 80% Acetonitril, 20% Wasser und 0,1% Ameisensäure verdünnt und extrahiert. Proben wurden bei 1.25 h, 2.75 h, 4.25 h, 18.25 h, und 21.75 h nach Start der Biotransformation von allen Reaktoren genommen. Die Umsatzraten für Sauerstoff (OTR = oxygen transfer rate) und Kohlenstoff (CTR = carbon transfer rate) wurden während der Fermentation über die Abgasanalytik an den DASGIP-Systemen bestimmt. Die Fermentation wurde 21.75 h nach Start der Biotransformation beendet. Der Rührer, die Begasung, die Temperaturregelung und pH-Regelung wurden ausgestellt und die Kessel 5-10 Minuten ruhig stehen gelassen.

### HPLC-ESI/MS-Scan-Methode

Die qualitative Beurteilung der Proben erfolgte mittels HPLC/MS-Kopplung mit hochaufgelöster MS-Detektion im Scan-Modus.

Dabei kamen folgende Geräte zum Einsatz:
- HPLC Anlage Accela (Thermo Scientific, Waltham, Massachusetts, USA) mit Autosampler, quaternärer Pumpe, PDA-Detektor und Säulenofen
- Massenspektrometer LTQ-FT (Thermo Scientific, Waltham, Massachusetts, USA) mit ESI-Quelle
- HPLC-Säule: Kinetex C18, 100 x 2,1 mm, Partikelgröße: 2,6 µm, Porengröße 100 Å (Phenomenex; Aschaffenburg)

Die Proben wurden vorbereitet, indem 1950 µL Lösungsmittel (80% (v/v) Acetonitril, 20% bidest. H₂O (v/v), + 0.1% Ameisensäure) und 50 µL Probe in ein 2-mL-Reaktionsgefäß pipettiert wurden. Das Gemisch wurde ca. 10 Sekunden gevortext und anschließend bei ca. 13000 rpm für 5 min zentrifugiert. Der klare Überstand wurde mit einer Pipette entnommen.

Die HPLC-Trennung erfolgte mit oben genannter HPLC-Säule. Das Injektionsvolumen betrug 0,5 µL, die Säulentemperatur 40°C und die Flussrate 0,3 mL/min. Die mobile Phase setzte sich zusammen aus Eluent A (0,02 %ige (v/v) wässrige Trifluoressigsäure) und Eluent B (Acetonitril mit 0,015 % (v/v) Trifluoressigsäure). Folgendes Gradientenprofil wurde genutzt:

| **Zeit [min]** | **Eluent A [%]** | **Eluent B [%]** |
|---|---|---|
| 0 | 98 | 2 |
| 2 | 98 | 2 |
| 17 | 2 | 98 |
| 32 | 2 | 98 |

Die ESI-MS-Analyse erfolgte im positiven Modus mit folgenden Parametern der ESI-Quelle:
- ESI-Spannung: 4kV
- Kapillartemperatur 300°C
- Sheath Gas Flow 40
- Aux Gas Flow 5
- Sweep Gas Flow 3

Die Detektion erfolgte in einem Massenbereich von m/z = 100 bis 1000. Die massenspekrometrische Auflösung betrug R = 100.000.

Die Ergebnisse sind in der folgenden Tabelle dargestellt.

| | | Intensität MS [-] | | |
|---|---|---|---|---|
| Substrat | Induktion | Dodekandiamin | Dodecylamin | Oleylamin |
| LS/ Ölsäure (150 ml) | 1 mM IPTG (12h) | n.n. | 1.160.000 | 23.700 |
| DDS/ Ölsäure (75 ml) | 1 mM IPTG in H₂O/ Ethanol (12h) | 742.000 | n.n. | 18.000 |
| DDS/ Ölsäure (150 ml) | 1 mM IPTG (1h) | 30.700 | n.n. | n.n. |
| DDS/ Ölsäure (150 ml) | 1 mM IPTG (12h) | 23.500 | n.n. | n.n. |

Qualitativer Nachweis von Mono- und Diaminen in der Fermentationsbrühe des Stammes *E.coli* W3110 pACYC{Placuv5}[carA_Ms-npt_Noc] / pJ281_alaDH_B.s._TA_C.v.(ct) nach 21,75h Prozesszeit (n.n. = nicht nachweisbar, LS = Laurinsäure, DDS = Dodekandisäure).

Weitere Daten verdeutlicht Abbildung 1.

Die quantitative Bestimmung von 1,12-Dodekandiamin und Dodecylamin erfolgte mittels HPLC/UV Messung, nach Derivatisierung mittels ortho-Phthaldialdehyd. Es wurde der methanolische Überstand vermessen. Die wichtigsten chromatographischen Parameter sind in der nachfolgenden Tabelle zusammen gefasst.

| | |
|---|---|
| Säule | Luna 5u C8, 100 Å, 150 x 4.60 mm, (Phenomenex; Aschaffenburg) |
| HPLC Anlage | Agilent 1200 |
| Laufmittel A | 2.5 mL Essigsäure (100 %) auf 1 L bidest. Wasser, pH Einstellung mit Natronlauge auf pH 6.0 |
| Laufmittel B | Methanol |
| Säulentemp. | 40 °C |
| Fluss | 1 mL/min |
| Gradient | 0.0-1 min: 30.0% B, 1.0-17.0 min: 90.0% B, 17-19.5 min: 90.0% B, 19.6-20.5 min: 30.0% B |
| Detektor | DAD, 334 nm |
| Derivatisierung/ Injektionsvolumen | Automatische Derivatisierung mittels Injektorprogramm, 1 µL Probe wird mit 9 µL Derivatisierungsreagenz umgesetzt; Zusammensetzung Derivatisierungsreagenz: 10 g/L o-Phthaldialdehyd gelöst in Boratpuffer (0.4 mol/L), unter Zugabe von Mercaptoethanol (5 mL/L) und Methanol (100 mL/L) |
| Kalibrierung | Externe Kalibrierung, Messbereich 50-1000 mg/L, 5-Punkte Kalibrierung, Kalibrierung vor und nach der Probenserie, Mittelung über beiden Kalibrierreihen, quadratische Regression |

Die Ergebnisse sind in den folgenden Tabellen dargestellt.

| Substrat | Induktion | Dodecylamin [mg/L] | Dodekandiamin [mg/L] |
|---|---|---|---|
| DDS/ Ölsäure (75 ml) | 1 mM IPTG in H₂O/Ethanol (12h) | n.n. | 40,5 |
| DDS/Ölsäure (150 mL) | 1 mM IPTG (1h) | n.n. | 3,1 |
| DDS/ Ölsäure (150 ml) | 1 mM IPTG (12h) | n.n. | <1*) |
| LS/Ölsäure (150 ml) | 1 mM IPTG (12h) | 11,6 | n.n. |

Quantifizierung von Mono- und Diaminen in der Fermentationsbrühe des Stammes *E.coli* W3110 pACYC{Placuv5}[carA_Ms-npt_Noc] / pJ281_alaDH_B.s._TA_C.v.(ct) nach 21,75h Prozesszeit (n.n. = nicht nachweisbar, *⁾ kleiner als Bestimmungsgrenze, DDS = Dodekandisäure, LS = Laurinsäure).

Es wurde gezeigt, dass die Stämme in der Lage sind aus Dodekansäure, Dodekandisäure und Ölsäure die jeweiligen Amine Dodecylamin, Dodekandiamin und Oleylamin herzustellen.

### Beispiel 5

### Herstellung eines Expressionsvektors für die Expression des Gens αDOX codierend für eine α-Dioxygenase aus Oryza sativa

Zur Herstellung eines Vektors für die Expression von αDOX (Os12g0448900, SEQ ID Nr. 16) aus *Oryza sativa* kodierend für eine α-Dioxygenase (NP_001066718.1) wurde das Gen für die Expression in *Escherichia coli* codonoptimiert, synthetisiert und gleichzeitig eine *Nde*I-Schnittstelle stromaufwärts und eine *Avr*II-Schnittstelle stromabwärts eingeführt. Das synthetisierte DNA-Fragment wurde mit den Restriktionsendonukleasen *Nde*I und *Avr*II verdaut und in den entsprechend geschnittenen Vektor pACYC{Placuv5}[carA_Ms-npt_Noc] (SEQ ID Nr. 10) unter Entfernung der Gene carA_Ms und npt_Noc ligiert. Der in dem Vektor enthaltene lacuv5-Promotor (SEQ ID Nr. 3) blieb dabei erhalten. Der fertiggestellte Vektor wurde als pACYC{Placuv5}[DOX_Os(co_Ec)] (SEQ ID Nr. 17) bezeichnet. Der Vektor pACYC ist ein *E.coli*-Vektor, der Chloramphenicol-Resistenz vermittelt sowie einen p15A Replikationsursprung trägt und damit eine niedrige Kopienzahl (10-15 Kopien pro Zelle) aufweist.

### Beispiel 6

### Herstellung eines E.coli-Stammes mit Deletion im Gen bioH, der die Gene αDOX aus Oryza sativa, ald aus Bacillus subtilis und Cv2025 aus Chromobacterium violaceum überexprimiert

Zur Erzeugung eines *E.coli*-Stammes, welcher das Gen αDOX aus *Oryza sativa* kodierend für eine α-Dioxygenase (NP_001066718.1) in Kombination mit den Genen *ald* (SEQ ID Nr. 11) aus *Bacillus subtilis* kodierend für eine Alanindehydrogenase (NP_391071.1) und Cv2025 (SEQ ID Nr. 12) aus *Chromobacterium violaceum* kodierend für eine Transaminase (NP_901695.1) coexprimiert wurde der Stamm *E.coli* W3110 Δ*bioH* (Herstellung siehe EP12007663, Beispiel 1) mit den Plasmiden pACYC{Placuv5}[DOX_Os(co_Ec)] (SEQ ID Nr. 17) und pJ281_alaDH_B.s._TA_C.v.(ct) (SEQ ID Nr. 15) mittels Elektroporation transformiert und auf LB-Agar-Platten mit Chloramphenicol (50 µg/ml) und Kanamycin (50 µg/ml) ausplattiert. Transformanten wurden durch Plasmidpräparation und analytische Restriktionsanalyse bezüglich der Präsenz der korrekten Plasmide überprüft. Der erzeugte Stamm wurde als *E.coli* ΔbioH pACYC{Placuv5}[DOX_Os(co_Ec)] / pJ281_alaDH_B.s._TA_C.v.(ct) bezeichnet.

Der Stamm wurde verwendet um seine Fähigkeit zur Produktion von Aminoundecansäuremethylester ausgehend von Dodecandisäuremethylester zu untersuchen.

### Beispiel 7

### Produktion von Aminoundecansäuremethylester ausgehend von Dodecandisäuremethylester durch einen E.coli Stamm mit einem Expressionsvektor für das Gen αDOX aus Oryza sativa in Kombination mit einem Expressionsvektor für die Gene ald aus Bacillus subtilis und Cv_2025 aus Chromobacterium violaceum

Der in Beispiel 8 beschriebene Stamm wurde verwendet, um seine Fähigkeit zur Produktion von Aminoundecansäuremethylester zu untersuchen. Dabei wurde wie folgt vorgegangen:
Der zu untersuchende Stamm wurde zunächst auf einer LB-Agarplatte mit 50 µg/ml Chloramphenicol und 50 µg/ml Kanamycin ausgestrichen und bei 37°C über Nacht inkubiert. Als Kontrolle wurde zusätzlich der Stamm *E.coli* W3110 ΔbioH auf einer LB-Agarplatte ohne Antibiotika ausgestrichen. Die Stämme wurden dann in Luria-Bertani Bouillon nach Miller (Merck, Darmstadt) mit 50 µg/ml Chloramphenicol und 50 µg/ml Kanamycin (für den plasmidtragenden Stamm) als 20 ml-Vorkultur aus je einer Einzelkolonie angezogen. Als Hauptkultur wurden 100 ml LB-Bouillon mit mit 50 µg/ml Chloramphenicol und 50 µg/ml Kanamycin in einen 500 ml-Erlenmeyerkolben mit Schikane vorgelegt und mit 2 ml aus der Vorkultur inokuliert. Die Kultivierung erfolgte zunächst bei 37°C und 200 U/min in einem Inkubationsschüttler. Bei Erreichen einer optischen Dichte (600 nm) von 0,5 - 0,7 wurde die Genexpression durch Zugabe von 1 mM IPTG induziert. Die weitere Kultivierung erfolgte bei 22°C und 200 U/min über Nacht. Am darauffolgenden Tag wurden die Kulturen durch 10minütige Zentrifugation bei 4°C und 5525 x g geerntet. Der Überstand wurde verworfen und das Zellpellet in 200 mM Kaliumphosphatpuffer (pH 7,5) gewaschen. Das Zellpellet wurde schließlich in 200 mM Kaliumphosphatpuffer mit 50 mM Ammoniumchlorid und 0,5% (w/v) Glucose aufgenommen, so dass eine OD (600 nm) von 20 erreicht wurde. Zu der Zellsuspension wurden 12,5 mM Dodecandisäuremethylester (abcr, Karlsruhe) in Ethanol zugegeben und 4 Stunden bei 30°C und 300 rpm leicht geschüttelt. Während der Inkubation wurden zu den Zeitpunkten 0 min, 60 min, 120 min, 180 min und 240 min Proben genommen und in einem Gemisch aus 80% Acetonitril, 20% Wasser und 0,1% Ameisensäure extrahiert. Der Überstand wurde mittels HPLC/MS-Analytik analysiert. Die Ergebnisse sind in der folgenden Tabelle dargestellt.

| Stamm | Zeit [min] | Peakfläche Aminoundecansäuremethylester |
|---|---|---|
| *E.coli* W3110 ΔbioH | 0 | n.n. |
| | 120 | n.n. |
| | 180 | n.n. |
| | 240 | n.n. |
| *E.coli* W3110 ΔbioH pACYC{Placuv5}[DOX] / pJ281_alaDH_B.s._TA_C.v.(ct) | 0 | n.n. |
| | 120 | 57616 |
| | 180 | 371989 |
| | 240 | 1764605 |

Produktion von Aminoundecansäuremethylester mit *E.coli* W3110 ΔbioH, der αDOX aus *Oryza sativa, ald* aus *Bacillus subtilis* und Cv_2025 aus *Chromobacterium violaceum* überexprimiert. Angegeben sind Peakflächen (n.n. = nicht nachweisbar).

Es konnte gezeigt werden, dass der Stamm *E.coli* W3110 ΔbioH pACYC{Placuv5}[DOX] / pJ281_alaDH_B.s._TA_C.v.(ct) zur Bildung von Aminoundecansäuremethylester ausgehend von Dodecandisäuremethylester fähig ist.

## Patentansprüche

1. Ganzzellkatalysator, der eine rekombinante α-Dioxygenase oder die Kombination aus einer rekombinanten Fettsäurereduktase und aus einer die Fettsäurereduktase phosphopantheteinylierenden Phosphopantheteinyl-Transferase exprimiert, und der zusätzlich eine Transaminase exprimiert.

2. Ganzzellkatalysator nach Anspruch 1, wobei der Ganzzellkatalysator zusätzlich eine Aminosäuredehydrogenase exprimiert.

3. Ganzzellkatalysator nach einem der Ansprüche 1 bis 2, wobei der Ganzzellkatalysator zusätzlich eine Alkanhydroxylase exprimiert.

4. Ganzzellkatalysator nach einem der Ansprüche 1 bis 3, wobei der Ganzzellkatalysator zusätzlich ein Polypeptid der AlkL-Familie exprimiert.

5. Ganzzellkatalysator nach einem der Ansprüche 1 bis 4, der zusätzlich eine Alkoholdehydrogenase exprimiert.

6. Ganzzellkatalysator nach einem der Ansprüche 1 bis 5, wobei die Aktivität wenigstens eines an der β-Oxidation beteiligten Enzyms gegenüber dem Wildtyp des Ganzzellkatalysators verringert ist.

7. Ganzzellkatalysator nach einem der Ansprüche 1 bis 6, wobei die Aktivität von BioH oder einer Variante davon gegenüber dem Wildtyp des Ganzzellkatalysators verringert oder erhöht ist.

8. Ganzzellkatalysator nach einem der Ansprüche 1 bis 7, wobei die Aktivität von FadL oder einer Variante davon gegenüber dem Wildtyp des Ganzzellkatalysators erhöht ist.

9. Verfahren zur Umsetzung einer Carbonsäure oder Dicarbonsäure oder eines Monoesters davon zu einem Amin oder Diamin, umfassend die Schritte
a) Bereitstellen einer Carbonsäure oder Dicarbonsäure oder eines Monoesters davon, für den Fall, dass es sich um eine Dicarbonsäure handelt,
b) Kontaktieren des Carbonsäure oder Dicarbonsäure oder des Monoesters davon mit einer phosphopantheteinylierten Fettsäurereduktase oder einer α-Dioxygenase unter Bildung eines Aldehydproduktes, und
c) Kontaktieren des Aldehydproduktes aus Schritt b) mit einer Transaminase.

10. Verfahren nach Anspruch 9, wobei in Schritt c) eine Aminosäuredehydrogenase anwesend ist.

11. Verfahren nach einem der Ansprüche 9 bis 10, wobei wenigstens ein Enzym aus der Gruppe umfassend phosphopantheteinylierte Fettsäurereduktase, α-Dioxygenase, Transaminase, Aminosäuredehydrogenase und Alkanhydroxylase in Form eines Ganzzellkatalysators nach einem der Ansprüche 1 bis 8 bereitgestellt werden.

12. Verfahren nach einem der Ansprüche 9 bis 11, wobei es sich bei der Carbonsäure oder Dicarbonsäure oder dem Monoester davon um eine Verbindung der Formel (I)
R1-A-COOR² (I)
handelt, wobei R¹ aus der Gruppe ausgewählt ist, die -H und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoff darstellt.

13. Verfahren nach einem der Ansprüche 9 bis 12, wobei A die Formel - (CH₂)ₙ - aufweist, wobei n wenigstens 4, bevorzugt wenigstens 10 ist.

14. Verwendung des Ganzzellkatalysators nach einem der Ansprüche 1 bis 8 oder des Verfahrens nach einem der Ansprüche 9 bis 12 zur Aminierung einer Carbonsäure oder Dicarbonsäure oder eines Monoesters davon.

15. Reaktionsmischung umfassend den Ganzzellkatalysator nach einem der Ansprüche 1 bis 8 in wässriger Lösung sowie eine Carbonsäure oder Dicarbonsäure oder einen Monoester davon der Formel (I)
R¹ - A - COOR² (I),
wobei R¹ aus der Gruppe ausgewählt ist, die -H und COOR³ umfasst,
wobei R² und R³ jeweils und unabhängig voneinander aus der Gruppe ausgewählt sind, die H, Methyl, Ethyl und Propyl umfasst,
mit der Maßgabe, dass wenigstens einer der Reste R² und R³ H ist,
wobei A eine unverzweigte, verzweigte, lineare, zyklische, substituierte oder unsubstituierte Kohlenwasserstoffgruppe mit wenigstens vier Kohlenstoff darstellt.

## Claims

1. Whole-cell catalyst which expresses a recombinant α-dioxygenase or the combination of a recombinant fatty acid reductase and of a phosphopantetheinyl transferase which phosphopantetheinylates the fatty acid reductase, and which additionally expresses a transaminase.

2. Whole-cell catalyst according to Claim 1, wherein the whole-cell catalyst additionally expresses an amino acid dehydrogenase.

3. Whole-cell catalyst according to either of Claims 1 and 2, wherein the whole-cell catalyst additionally expresses an alkane hydroxylase.

4. Whole-cell catalyst according to any of Claims 1 to 3, wherein the whole-cell catalyst additionally expresses a polypeptide of the AlkL family.

5. Whole-cell catalyst according to any of Claims 1 to 4, which additionally expresses an alcohol dehydrogenase.

6. Whole-cell catalyst according to any of Claims 1 to 5, wherein the activity of at least one enzyme involved in the β-oxidation is reduced with respect to the wild type of the whole-cell catalyst.

7. Whole-cell catalyst according to any of Claims 1 to 6, wherein the activity of BioH or a variant thereof is reduced or elevated with respect to the wild type of the whole-cell catalyst.

8. Whole-cell catalyst according to any of Claims 1 to 7, wherein the activity of FadL or a variant thereof is elevated with respect to the wild type of the whole-cell catalyst.

9. Process for converting a carboxylic acid or dicarboxylic acid or a monoester thereof to an amine or diamine, comprising the steps of
a) providing a carboxylic acid or dicarboxylic acid or a monoester thereof, in the case of a dicarboxylic acid,
b) contacting the carboxylic acid or dicarboxylic acid or the monoester thereof with a phosphopantetheinylated fatty acid reductase or an α-dioxygenase to form an aldehyde product, and
c) contacting the aldehyde product from step b) with a transaminase.

10. Process according to Claim 9, wherein an amino acid dehydrogenase is present in step c).

11. Process according to any of Claims 9 to 10, wherein at least one enzyme from the group comprising phosphopantetheinylated fatty acid reductase, α-dioxygenase, transaminase, amino acid dehydrogenase and alkane hydroxylase, are provided in the form of a whole-cell catalyst according to any of Claims 1 to 8.

12. Process according to any of Claims 9 to 11, wherein the carboxylic acid or dicarboxylic acid or the monoester thereof is a compound of the formula (I)
R¹ - A - COOR² (I),
where R¹ is selected from the group comprising -H and COOR³,
where R² and R³ are each independently selected from the group comprising H, methyl, ethyl and propyl,
with the proviso that at least one of the radicals R² and R³ is H,
where A is an unbranched, branched, linear, cyclic, substituted or unsubstituted hydrocarbon group having at least four carbons.

13. Process according to any of Claims 9 to 12, wherein A has the formula - (CH₂)ₙ -, where n is at least 4, preferably at least 10.

14. Use of the whole-cell catalyst according to any of Claims 1 to 8 or of the process according to any of Claims 9 to 12 for aminating a carboxylic acid or dicarboxylic acid or a monoester thereof.

15. Reaction mixture comprising the whole-cell catalyst according to any of Claims 1 to 8 in aqueous solution and also a carboxylic acid or dicarboxylic acid or a monoester thereof of the formula (I)
R¹ - A - COOR² (I),
where R¹ is selected from the group comprising -H and COOR³,
where R² and R³ are each independently selected from the group comprising H, methyl, ethyl and propyl,
with the proviso that at least one of the radicals R² and R³ is H,
where A is an unbranched, branched, linear, cyclic, substituted or unsubstituted hydrocarbon group having at least four carbons.

## Revendications

1. Catalyseur à cellules entières, qui exprime une α-dioxygénase recombinante ou la combinaison d'une acide gras réductase recombinante et d'une phosphopantéthéinyl-transférase phosphopantéthéinylisant l'acide gras réductase et qui exprime en outre une transaminase.

2. Catalyseur à cellules entières selon la revendication 1, le catalyseur à cellules entières exprimant en outre une acide aminé déshydrogénase.

3. Catalyseur à cellules entières selon l'une quelconque des revendications 1 et 2, le catalyseur à cellules entières exprimant en outre une alcane hydroxylase.

4. Catalyseur à cellules entières selon l'une quelconque des revendications 1 à 3, le catalyseur à cellules entières exprimant en outre un polypeptide de la famille des AlkL.

5. Catalyseur à cellules entières selon l'une quelconque des revendications 1 à 4, qui exprime en outre une alcool déshydrogénase.

6. Catalyseur à cellules entières selon l'une quelconque des revendications 1 à 5, l'activité d'au moins une enzyme participant à la β-oxydation étant diminuée par rapport à celle du type sauvage du catalyseur à cellules entières.

7. Catalyseur à cellules entières selon l'une quelconque des revendications 1 à 6, l'activité de BioH ou d'une variante de celle-ci étant diminuée ou augmentée par rapport à celle du type sauvage du catalyseur à cellules entières.

8. Catalyseur à cellules entières selon l'une quelconque des revendications 1 à 7, l'activité de FadL ou d'une variante de celle-ci étant augmentée par rapport à celle du type sauvage du catalyseur à cellules entières.

9. Procédé pour la transformation d'un acide carboxylique ou dicarboxylique ou d'un monoester correspondant en une amine ou en une diamine, comprenant les étapes de
a) mise à disposition d'un acide carboxylique ou dicarboxylique ou d'un monoester correspondant, pour le cas où il s'agit d'un acide dicarboxylique,
b) mise en contact de l'acide carboxylique ou dicarboxylique ou du monoester correspondant avec une acide gras réductase phosphopantéthéinylisée ou une α-dioxygénase avec formation d'un produit de type aldéhyde et
c) mise en contact du produit de type aldéhyde de l'étape b) avec une transaminase.

10. Procédé selon la revendication 9, une acide aminé déshydrogénase étant présente dans l'étape c).

11. Procédé selon l'une quelconque des revendications 9 et 10, au moins une enzyme du groupe comprenant une acide gras réductase phosphopantéthéinylisée, une α-dioxygénase, une transaminase, une acide aminé déshydrogénase et une alcane hydroxylase étant préparée sous forme d'un catalyseur à cellules entières selon l'une quelconque des revendications 1 à 8.

12. Procédé selon l'une quelconque des revendications 9 à 11, où il s'agit, pour l'acide carboxylique ou dicarboxylique ou le monoester correspondant, d'un composé de formule (I)
R¹-A-COOR² (I)
dans laquelle R¹ est choisi dans le groupe comprenant -H et COOR³,
R² et R³ étant choisis, à chaque fois et indépendamment l'un de l'autre, dans le groupe comprenant H, méthyle, éthyle et propyle,
à condition qu'au moins un des radicaux R² et R³ représente H,
A représentant un groupe hydrocarboné non ramifié, ramifié, linéaire, cyclique, substitué ou non substitué comprenant au moins quatre atomes de carbone.

13. Procédé selon l'une quelconque des revendications 9 à 12, A présentant la formule -(CH₂)ₙ-, dans laquelle n vaut au moins 4, de préférence au moins 10.

14. Utilisation du catalyseur à cellules entières selon l'une quelconque des revendications 1 à 8 ou du procédé selon l'une quelconque des revendications 9 à 12 pour l'amination d'un acide carboxylique ou dicarboxylique ou d'un monoester correspondant.

15. Mélange réactionnel comprenant le catalyseur à cellules entières selon l'une quelconque des revendications 1 à 8 en solution aqueuse ainsi qu'un acide carboxylique ou dicarboxylique ou un monoester correspondant de formule (I)
R¹-A-COOR² (I),
dans laquelle R¹ est choisi dans le groupe comprenant -H et COOR³,
R² et R³ étant choisis, à chaque fois et indépendamment l'un de l'autre, dans le groupe comprenant H, méthyle, éthyle et propyle,
à condition qu'au moins un des radicaux R² et R³ représente H,
A représentant un groupe hydrocarboné non ramifié, ramifié, linéaire, cyclique, substitué ou non substitué comprenant au moins quatre atomes de carbone.


### SEQUENCE LISTING

<110> Evonik Industries AG
<120> Herstellung von Aminen und Diaminen aus einer Carbonsäure oder Dicarbonsäure oder eines Monoesters davon
<130> 2012000206
<160> 17
<170> PatentIn version 3.5
<210> 1
   <211> 3507
   <212> DNA
   <213> Mycobacterium smegmatis
<400> 1
   atgacgatcg aaacgcgcga agaccgcttc aaccggcgca ttgaccactt gttcgaaacc      60
   gacccgcagt tcgccgccgc ccgtcccgac gaggcgatca gcgcggctgc cgccgatccg     120
   gagttgcgcc ttcctgccgc ggtcaaacag attctggccg gctatgcgga ccgccctgcg     180
   ctcggcaagc gcgccgtcga gttcgtcacc gacgaagaag gccgcaccac cgcgaagctc     240
   ctgccccgct tcgacaccat cacctaccgt cagctcgcag gccggatcca ggccgtgacc     300
   aatgcctggc acaaccatcc ggtgaatgcc ggtgaccgcg tggccatcct gggtttcacc     360
   agtgtcgact acacgacgat cgacatcgcc ctgctcgaac tcggcgccgt gtccgtaccg     420
   ctgcagacca gtgcgccggt ggcccaactg cagccgatcg tcgccgagac cgagcccaag     480
   gtgatcgcgt cgagcgtcga cttcctcgcc gacgcagtcg ctctcgtcga gtccgggccc     540
   gcgccgtcgc gactggtggt gttcgactac agccacgagg tcgacgatca gcgtgaggcg     600
   ttcgaggcgg ccaagggcaa gctcgcaggc accggcgtcg tcgtcgagac gatcaccgac     660
   gtactggacc gcgggcggtc actcgccgac gcaccgctct acgtgcccga cgagaccgac     720
   ccgctgaccc ttctcatcta cacctccggc agcaccggca ctcccaaggg cgcgatgtac     780
   cccgagtcca agaccgccac gatgtggcag gccgggtcca aggcccggtg ggacgagacc     840
   ctcggcgtga tgccgtcaat caccctgaac ttcatgccca tgagtcacgt catggggcgc     900
   ggcatcctgt gcagcacact cgccagcggc ggaaccgcgt acttcgccgc acgcagcgac     960
   ctgtccacct tcctggagga cctcgccctc gtgcggccca cgcagctcaa cttcgttcct    1020
   cgcatctggg acatgctgtt ccaggagtac cagagccgcc tcgacaaccg ccgcgccgag    1080
   ggatccgagg accgagccga agccgcagtc ctcgaagagg tccgcaccca actgctcggc    1140
   gggcgattcg tttcggccct gaccggatcg gctcccatct cggcggagat gaagagctgg    1200
   gtcgaggacc tgctcgacat gcatctgctg gagggctacg gctccaccga ggccggcgcg    1260
   gtgttcatcg acgggcagat ccagcgcccg ccggtcatcg actacaagct ggtcgacgtg    1320
   cccgatctcg gctacttcgc cacggaccgg ccctacccgc gcggcgaact tctggtcaag 1380
   tccgagcaga tgttccccgg ctactacaag cgtccggaga tcaccgccga gatgttcgac 1440
   gaggacgggt actaccgcac cggcgacatc gtcgccgagc tcgggcccga ccatctcgaa 1500
   tacctcgacc gccgcaacaa cgtgctgaaa ctgtcgcagg gcgaattcgt cacggtctcc 1560
   aagctggagg cggtgttcgg cgacagcccc ctggtacgcc agatctacgt ctacggcaac 1620
   agcgcgcggt cctatctgct ggcggtcgtg gtcccgaccg aagaggcact gtcacgttgg 1680
   gacggtgacg aactcaagtc gcgcatcagc gactcactgc aggacgcggc acgagccgcc 1740
   ggattgcagt cgtatgagat cccgcgtgac ttcctcgtcg agacaacacc tttcacgctg 1800
   gagaacggcc tgctgaccgg tatccgcaag ctggcccggc cgaaactgaa ggcgcactac 1860
   ggcgaacgcc tcgaacagct ctacaccgac ctggccgagg ggcaggccaa cgagttgcgc 1920
   gagttgcgcc gcaacggagc cgaccggccc gtggtcgaga ccgtcagccg cgccgcggtc 1980
   gcactgctcg gtgcctccgt cacggatctg cggtccgatg cgcacttcac cgatctgggt 2040
   ggagattcgt tgtcggcctt gagcttctcg aacctgttgc acgagatctt cgatgtcgac 2100
   gtgccggtcg gcgtcatcgt cagcccggcc accgacctgg caggcgtcgc ggcctacatc 2160
   gagggcgaac tgcgcggctc caagcgcccc acatacgcgt cggtgcacgg gcgcgacgcc 2220
   accgaggtgc gcgcgcgtga tctcgccctg ggcaagttca tcgacgccaa gaccctgtcc 2280
   gccgcgccgg gtctgccgcg ttcgggcacc gagatccgca ccgtgctgct gaccggcgcc 2340
   accgggttcc tgggccgcta tctggcgctg gaatggctgg agcgcatgga cctggtggac 2400
   ggcaaggtga tctgcctggt gcgcgcccgc agcgacgacg aggcccgggc gcgtctggac 2460
   gccacgttcg acaccgggga cgcgacactg ctcgagcact accgcgcgct ggcagccgat 2520
   cacctcgagg tgatcgccgg tgacaagggc gaggccgatc tgggtctcga ccacgacacg 2580
   tggcagcgac tggccgacac cgtcgatctg atcgtcgatc cggccgccct ggtcaatcac 2640
   gtcctgccgt acagccagat gttcggaccc aatgcgctcg gcaccgccga actcatccgg 2700
   atcgcgctga ccaccacgat caagccgtac gtgtacgtct cgacgatcgg tgtgggacag 2760
   ggcatctccc ccgaggcgtt cgtcgaggac gccgacatcc gcgagatcag cgcgacgcgc 2820
   cgggtcgacg actcgtacgc caacggctac ggcaacagca agtgggccgg cgaggtcctg 2880
   ctgcgggagg cgcacgactg gtgtggtctg ccggtctcgg tgttccgctg cgacatgatc 2940
   ctggccgaca cgacctactc gggtcagctg aacctgccgg acatgttcac ccgcctgatg 3000
   ctgagcctcg tggcgaccgg catcgcgccc ggttcgttct acgaactcga tgcggacggc 3060
   aaccggcagc gcgcccacta cgacgggctg cccgtggagt tcatcgccga ggcgatctcc 3120
   accatcggct cgcaggtcac cgacggattc gagacgttcc acgtgatgaa cccgtacgac 3180
   gacggcatcg gcctcgacga gtacgtggac tggctgatcg aggccggcta ccccgtgcac 3240
   cgcgtcgacg actacgccac ctggctgagc cggttcgaaa ccgcactgcg ggccctgccg 3300
   gaacggcaac gtcaggcctc gctgctgccg ctgctgcaca actatcagca gccctcaccg 3360
   cccgtgtgcg gtgccatggc acccaccgac cggttccgtg ccgcggtgca ggacgcgaag 3420
   atcggccccg acaaggacat tccgcacgtc acggccgacg tgatcgtcaa gtacatcagc 3480
   aacctgcaga tgctcggatt gctgtaa 3507
<210> 2
   <211> 672
   <212> DNA
   <213> Nocardia sp.
<400> 2
   atgatcgaga caattttgcc tgctggtgtc gagtcggctg agctgctgga gtatccggag 60
   gacctgaagg cgcatccggc ggaggagcat ctcatcgcga agtcggtgga gaagcggcgc 120
   cgggacttca tcggggccag gcattgtgcc cggctggcgc tggctgagct cggcgagccg 180
   ccggtggcga tcggcaaagg ggagcggggt gcgccgatct ggccgcgcgg cgtcgtcggc 240
   agcctcaccc attgcgacgg atatcgggcc gcggcggtgg cgcacaagat gcgcttccgt 300
   tcgatcggca tcgatgccga gccgcacgcg acgctgcccg aaggcgtgct ggattcggtc 360
   agcctgccgc cggagcggga gtggttgaag accaccgatt ccgcactgca cctggaccgt 420
   ttactgttct gcgccaagga agccacctac aaggcgtggt ggccgctgac cgcgcgctgg 480
   ctcggcttcg aggaagcgca catcaccttc gagatcgaag acggctccgc cgattccggc 540
   aacggcacct ttcacagcga gctgctggtg ccgggacaga cgaatgacgg tgggacgccg 600
   ctgctttcgt tcgacggccg gtggctgatc gccgacgggt tcatcctcac cgcgatcgcg 660
   tacgcctgat aa 672
<210> 3
   <211> 153
   <212> DNA
   <213> Artificial Sequence
<220>
   <223> promotor
<400> 3
   ggcagtgagc gcaacgcaat taatgtaagt tagctcactc attaggcacc ccaggcttga 60
   cactttatgc ttccggctcg tataatgtgt ggaattgtga gcggataaca ataacaattt 120
   cacacaggat ctaggaacca aggagagtgg cat 153
<210> 4
   <211> 34
   <212> DNA
   <213> Artificial Sequence
<220>
   <223> Primer
<400> 4
   ttatgcgact cctgctggct atggtgggat ttcc 34
<210> 5
   <211> 29
   <212> DNA
   <213> Artificial Sequence
<220>
   <223> Primer
<400> 5
   gatcgtcata tgccactctc cttggttcc 29
<210> 6
   <211> 25
   <212> DNA
   <213> Artificial Sequence
<220>
   <223> Primer
<400> 6
   tggcatatga cgatcgaaac gcgcg 25
<210> 7
   <211> 28
   <212> DNA
   <213> Artificial Sequence
<220>
   <223> Primer
<400> 7
   tccttctctt acagcaatcc gagcatct 28
<210> 8
   <211> 32
   <212> DNA
   <213> Artificial Sequence
<220>
   <223> Primer
<400> 8
   gctgtaagag aaggagttct atcatgatcg ag 32
<210> 9
   <211> 35
   <212> DNA
   <213> Artificial Sequence
<220>
   <223> Primer
<400> 9
   gcagcctagg ttaatttatc aggcgtacgc gatcg 35
<210> 10
   <211> 8047
   <212> DNA
   <213> Artificial Sequence
<220>
   <223> Vector
<400> 10
   attaacctag gctgctgcca ccgctgagca ataactagca taaccccttg gggcctctaa      60
   acgggtcttg aggggttttt tgctgaaacc tcaggcattt gagaagcaca cggtcacact     120
   gcttccggta gtcaataaac cggtaaacca gcaatagaca taagcggcta tttaacgacc     180
   ctgccctgaa ccgacgaccg ggtcgaattt gctttcgaat ttctgccatt catccgctta     240
   ttatcactta ttcaggcgta gcaccaggcg tttaagggca ccaataactg ccttaaaaaa     300
   attacgcccc gccctgccac tcatcgcagt actgttgtaa ttcattaagc attctgccga     360
   catggaagcc atcacagacg gcatgatgaa cctgaatcgc cagcggcatc agcaccttgt     420
   cgccttgcgt ataatatttg cccatagtga aaacgggggc gaagaagttg tccatattgg     480
   ccacgtttaa atcaaaactg gtgaaactca cccagggatt ggctgagacg aaaaacatat     540
   tctcaataaa ccctttaggg aaataggcca ggttttcacc gtaacacgcc acatcttgcg     600
   aatatatgtg tagaaactgc cggaaatcgt cgtggtattc actccagagc gatgaaaacg     660
   tttcagtttg ctcatggaaa acggtgtaac aagggtgaac actatcccat atcaccagct     720
   caccgtcttt cattgccata cggaactccg gatgagcatt catcaggcgg gcaagaatgt     780
   gaataaaggc cggataaaac ttgtgcttat ttttctttac ggtctttaaa aaggccgtaa     840
   tatccagctg aacggtctgg ttataggtac attgagcaac tgactgaaat gcctcaaaat     900
   gttctttacg atgccattgg gatatatcaa cggtggtata tccagtgatt tttttctcca     960
   ttttagcttc cttagctcct gaaaatctcg ataactcaaa aaatacgccc ggtagtgatc    1020
   ttatttcatt atggtgaaag ttggaacctc ttacgtgccg atcaacgtct cattttcgcc    1080
   aaaagttggc ccagggcttc ccggtatcaa cagggacacc aggatttatt tattctgcga    1140
   agtgatcttc cgtcacaggt atttattcgg cgcaaagtgc gtcgggtgat gctgccaact    1200
   tactgattta gtgtatgatg gtgtttttga ggtgctccag tggcttctgt ttctatcagc    1260
   tgtccctcct gttcagctac tgacggggtg gtgcgtaacg gcaaaagcac cgccggacat    1320
   cagcgctagc ggagtgtata ctggcttact atgttggcac tgatgagggt gtcagtgaag    1380
   tgcttcatgt ggcaggagaa aaaaggctgc accggtgcgt cagcagaata tgtgatacag    1440
   gatatattcc gcttcctcgc tcactgactc gctacgctcg gtcgttcgac tgcggcgagc    1500
   ggaaatggct tacgaacggg gcggagattt cctggaagat gccaggaaga tacttaacag 1560
   ggaagtgaga gggccgcggc aaagccgttt ttccataggc tccgcccccc tgacaagcat 1620
   cacgaaatct gacgctcaaa tcagtggtgg cgaaacccga caggactata aagataccag 1680
   gcgtttcccc tggcggctcc ctcgtgcgct ctcctgttcc tgcctttcgg tttaccggtg 1740
   tcattccgct gttatggccg cgtttgtctc attccacgcc tgacactcag ttccgggtag 1800
   gcagttcgct ccaagctgga ctgtatgcac gaaccccccg ttcagtccga ccgctgcgcc 1860
   ttatccggta actatcgtct tgagtccaac ccggaaagac atgcaaaagc accactggca 1920
   gcagccactg gtaattgatt tagaggagtt agtcttgaag tcatgcgccg gttaaggcta 1980
   aactgaaagg acaagttttg gtgactgcgc tcctccaagc cagttacctc ggttcaaaga 2040
   gttggtagct cagagaacct tcgaaaaacc gccctgcaag gcggtttttt cgttttcaga 2100
   gcaagagatt acgcgcagac caaaacgatc tcaagaagat catcttatta atcagataaa 2160
   atatttctag atttcagtgc aatttatctc ttcaaatgta gcacctgaag tcagccccat 2220
   acgatataag ttgtaattct catgttagtc atgccccgcg cccaccggaa ggagctgact 2280
   gggttgaagg ctctcaaggg catcggtcga gatcccggtg cctaatgagt gagctaactt 2340
   acattaattg cgttgcgctc actgcccgct ttccagtcgg gaaacctgtc gtgccagctg 2400
   cattaatgaa tcggccaacg cgcggggaga ggcggtttgc gtattgggcg ccagggtggt 2460
   ttttcttttc accagtgaga cgggcaacag ctgattgccc ttcaccgcct ggccctgaga 2520
   gagttgcagc aagcggtcca cgctggtttg ccccagcagg cgaaaatcct gtttgatggt 2580
   ggttaacggc gggatataac atgagctgtc ttcggtatcg tcgtatccca ctaccgagat 2640
   gtccgcacca acgcgcagcc cggactcggt aatggcgcgc attgcgccca gcgccatctg 2700
   atcgttggca accagcatcg cagtgggaac gatgccctca ttcagcattt gcatggtttg 2760
   ttgaaaaccg gacatggcac tccagtcgcc ttcccgttcc gctatcggct gaatttgatt 2820
   gcgagtgaga tatttatgcc agccagccag acgcagacgc gccgagacag aacttaatgg 2880
   gcccgctaac agcgcgattt gctggtgacc caatgcgacc agatgctcca cgcccagtcg 2940
   cgtaccgtct tcatgggaga aaataatact gttgatgggt gtctggtcag agacatcaag 3000
   aaataacgcc ggaacattag tgcaggcagc ttccacagca atggcatcct ggtcatccag 3060
   cggatagtta atgatcagcc cactgacgcg ttgcgcgaga agattgtgca ccgccgcttt 3120
   acaggcttcg acgccgcttc gttctaccat cgacaccacc acgctggcac ccagttgatc 3180
   ggcgcgagat ttaatcgccg cgacaatttg cgacggcgcg tgcagggcca gactggaggt 3240
   ggcaacgcca atcagcaacg actgtttgcc cgccagttgt tgtgccacgc ggttgggaat 3300
   gtaattcagc tccgccatcg ccgcttccac tttttcccgc gttttcgcag aaacgtggct 3360
   ggcctggttc accacgcggg aaacggtctg ataagagaca ccggcatact ctgcgacatc 3420
   gtataacgtt actggtttca cattcaccac cctgaattga ctctcttccg ggcgctatca 3480
   tgccataccg cgaaaggttt tgcgccattc gatggtgtcc gggatctcga cgctctccct 3540
   tatgcgactc ctgctggcta tggtgggatt tcccttgctg aaatgggaga tccgatcatg 3600
   ttcgagctct tattcaaata cactgctgtg ttggcggtaa gcgttctcga gcgcggccgc 3660
   gcgatcgcac ctggtgttta aacggccggc cctgcagggc gcagtgagcg caacgcaatt 3720
   aatgtaagtt agctcactca ttaggcaccc caggcttgac actttatgct tccggctcgt 3780
   ataatgtgtg gaattgtgag cggataacaa taacaatttc acacaggatc taggaaccaa 3840
   ggagagtggc atatgacgat cgaaacgcgc gaagaccgct tcaaccggcg cattgaccac 3900
   ttgttcgaaa ccgacccgca gttcgccgcc gcccgtcccg acgaggcgat cagcgcggct 3960
   gccgccgatc cggagttgcg ccttcctgcc gcggtcaaac agattctggc cggctatgcg 4020
   gaccgccctg cgctcggcaa gcgcgccgtc gagttcgtca ccgacgaaga aggccgcacc 4080
   accgcgaagc tcctgccccg cttcgacacc atcacctacc gtcagctcgc aggccggatc 4140
   caggccgtga ccaatgcctg gcacaaccat ccggtgaatg ccggtgaccg cgtggccatc 4200
   ctgggtttca ccagtgtcga ctacacgacg atcgacatcg ccctgctcga actcggcgcc 4260
   gtgtccgtac cgctgcagac cagtgcgccg gtggcccaac tgcagccgat cgtcgccgag 4320
   accgagccca aggtgatcgc gtcgagcgtc gacttcctcg ccgacgcagt cgctctcgtc 4380
   gagtccgggc ccgcgccgtc gcgactggtg gtgttcgact acagccacga ggtcgacgat 4440
   cagcgtgagg cgttcgaggc ggccaagggc aagctcgcag gcaccggcgt cgtcgtcgag 4500
   acgatcaccg acgtactgga ccgcgggcgg tcactcgccg acgcaccgct ctacgtgccc 4560
   gacgagaccg acccgctgac ccttctcatc tacacctccg gcagcaccgg cactcccaag 4620
   ggcgcgatgt accccgagtc caagaccgcc acgatgtggc aggccgggtc caaggcccgg 4680
   tgggacgaga ccctcggcgt gatgccgtca atcaccctga acttcatgcc catgagtcac 4740
   gtcatggggc gcggcatcct gtgcagcaca ctcgccagcg gcggaaccgc gtacttcgcc 4800
   gcacgcagcg acctgtccac cttcctggag gacctcgccc tcgtgcggcc cacgcagctc 4860
   aacttcgttc ctcgcatctg ggacatgctg ttccaggagt accagagccg cctcgacaac 4920
   cgccgcgccg agggatccga ggaccgagcc gaagccgcag tcctcgaaga ggtccgcacc 4980
   caactgctcg gcgggcgatt cgtttcggcc ctgaccggat cggctcccat ctcggcggag 5040
   atgaagagct gggtcgagga cctgctcgac atgcatctgc tggagggcta cggctccacc 5100
   gaggccggcg cggtgttcat cgacgggcag atccagcgcc cgccggtcat cgactacaag 5160
   ctggtcgacg tgcccgatct cggctacttc gccacggacc ggccctaccc gcgcggcgaa 5220
   cttctggtca agtccgagca gatgttcccc ggctactaca agcgtccgga gatcaccgcc 5280
   gagatgttcg acgaggacgg gtactaccgc accggcgaca tcgtcgccga gctcgggccc 5340
   gaccatctcg aatacctcga ccgccgcaac aacgtgctga aactgtcgca gggcgaattc 5400
   gtcacggtct ccaagctgga ggcggtgttc ggcgacagcc ccctggtacg ccagatctac 5460
   gtctacggca acagcgcgcg gtcctatctg ctggcggtcg tggtcccgac cgaagaggca 5520
   ctgtcacgtt gggacggtga cgaactcaag tcgcgcatca gcgactcact gcaggacgcg 5580
   gcacgagccg ccggattgca gtcgtatgag atcccgcgtg acttcctcgt cgagacaaca 5640
   cctttcacgc tggagaacgg cctgctgacc ggtatccgca agctggcccg gccgaaactg 5700
   aaggcgcact acggcgaacg cctcgaacag ctctacaccg acctggccga ggggcaggcc 5760
   aacgagttgc gcgagttgcg ccgcaacgga gccgaccggc ccgtggtcga gaccgtcagc 5820
   cgcgccgcgg tcgcactgct cggtgcctcc gtcacggatc tgcggtccga tgcgcacttc 5880
   accgatctgg gtggagattc gttgtcggcc ttgagcttct cgaacctgtt gcacgagatc 5940
   ttcgatgtcg acgtgccggt cggcgtcatc gtcagcccgg ccaccgacct ggcaggcgtc 6000
   gcggcctaca tcgagggcga actgcgcggc tccaagcgcc ccacatacgc gtcggtgcac 6060
   gggcgcgacg ccaccgaggt gcgcgcgcgt gatctcgccc tgggcaagtt catcgacgcc 6120
   aagaccctgt ccgccgcgcc gggtctgccg cgttcgggca ccgagatccg caccgtgctg 6180
   ctgaccggcg ccaccgggtt cctgggccgc tatctggcgc tggaatggct ggagcgcatg 6240
   gacctggtgg acggcaaggt gatctgcctg gtgcgcgccc gcagcgacga cgaggcccgg 6300
   gcgcgtctgg acgccacgtt cgacaccggg gacgcgacac tgctcgagca ctaccgcgcg 6360
   ctggcagccg atcacctcga ggtgatcgcc ggtgacaagg gcgaggccga tctgggtctc 6420
   gaccacgaca cgtggcagcg actggccgac accgtcgatc tgatcgtcga tccggccgcc 6480
   ctggtcaatc acgtcctgcc gtacagccag atgttcggac ccaatgcgct cggcaccgcc 6540
   gaactcatcc ggatcgcgct gaccaccacg atcaagccgt acgtgtacgt ctcgacgatc 6600
   ggtgtgggac agggcatctc ccccgaggcg ttcgtcgagg acgccgacat ccgcgagatc 6660
   agcgcgacgc gccgggtcga cgactcgtac gccaacggct acggcaacag caagtgggcc 6720
   ggcgaggtcc tgctgcggga ggcgcacgac tggtgtggtc tgccggtctc ggtgttccgc 6780
   tgcgacatga tcctggccga cacgacctac tcgggtcagc tgaacctgcc ggacatgttc 6840
   acccgcctga tgctgagcct cgtggcgacc ggcatcgcgc ccggttcgtt ctacgaactc 6900
   gatgcggacg gcaaccggca gcgcgcccac tacgacgggc tgcccgtgga gttcatcgcc 6960
   gaggcgatct ccaccatcgg ctcgcaggtc accgacggat tcgagacgtt ccacgtgatg 7020
   aacccgtacg acgacggcat cggcctcgac gagtacgtgg actggctgat cgaggccggc 7080
   taccccgtgc accgcgtcga cgactacgcc acctggctga gccggttcga aaccgcactg 7140
   cgggccctgc cggaacggca acgtcaggcc tcgctgctgc cgctgctgca caactatcag 7200
   cagccctcac cgcccgtgtg cggtgccatg gcacccaccg accggttccg tgccgcggtg 7260
   caggacgcga agatcggccc cgacaaggac attccgcacg tcacggccga cgtgatcgtc 7320
   aagtacatca gcaacctgca gatgctcgga ttgctgtaag agaaggagtt ctatcatgat 7380
   cgagacaatt ttgcctgctg gtgtcgagtc ggctgagctg ctggagtatc cggaggacct 7440
   gaaggcgcat ccggcggagg agcatctcat cgcgaagtcg gtggagaagc ggcgccggga 7500
   cttcatcggg gccaggcatt gtgcccggct ggcgctggct gagctcggcg agccgccggt 7560
   ggcgatcggc aaaggggagc ggggtgcgcc gatctggccg cgcggcgtcg tcggcagcct 7620
   cacccattgc gacggatatc gggccgcggc ggtggcgcac aagatgcgct tccgttcgat 7680
   cggcatcgat gccgagccgc acgcgacgct gcccgaaggc gtgctggatt cggtcagcct 7740
   gccgccggag cgggagtggt tgaagaccac cgattccgca ctgcacctgg accgtttact 7800
   gttctgcgcc aaggaagcca cctacaaggc gtggtggccg ctgaccgcgc gctggctcgg 7860
   cttcgaggaa gcgcacatca ccttcgagat cgaagacggc tccgccgatt ccggcaacgg 7920
   cacctttcac agcgagctgc tggtgccggg acagacgaat gacggtggga cgccgctgct 7980
   ttcgttcgac ggccggtggc tgatcgccga cgggttcatc ctcaccgcga tcgcgtacgc 8040
   ctgataa 8047
<210> 11
   <211> 1137
   <212> DNA
   <213> Bacillus subtilis
<400> 11
   atgatcatag gggttcctaa agagataaaa aacaatgaaa accgtgtcgc attaacaccc      60
   gggggcgttt ctcagctcat ttcaaacggc caccgggtgc tggttgaaac aggcgcgggc     120
   cttggaagcg gatttgaaaa tgaagcctat gagtcagcag gagcggaaat cattgctgat     180
   ccgaagcagg tctgggacgc cgaaatggtc atgaaagtaa aagaaccgct gccggaagaa     240
   tatgtttatt ttcgcaaagg acttgtgctg tttacgtacc ttcatttagc agctgagcct     300
   gagcttgcac aggccttgaa ggataaagga gtaactgcca tcgcatatga aacggtcagt     360
   gaaggccgga cattgcctct tctgacgcca atgtcagagg ttgcgggcag aatggcagcg     420
   caaatcggcg ctcaattctt agaaaagcct aaaggcggaa aaggcattct gcttgccggg     480
   gtgcctggcg tttcccgcgg aaaagtaaca attatcggag gaggcgttgt cgggacaaac     540
   gcggcgaaaa tggctgtcgg cctcggtgca gatgtgacga tcattgactt aaacgcagac     600
   cgcttgcgcc agcttgatga catcttcggc catcagatta aaacgttaat ttctaatccg     660
   gtcaatattg ctgatgctgt ggcggaagcg gatctcctca tttgcgcggt attaattccg     720
   ggtgctaaag ctccgactct tgtcactgag gaaatggtaa aacaaatgaa acccggttca     780
   gttattgttg atgtagcgat cgaccaaggc ggcatcgtcg aaactgtcga ccatatcaca     840
   acacatgatc agccaacata tgaaaaacac ggggttgtgc attatgctgt agcgaacatg     900
   ccaggcgcag tccctcgtac atcaacaatc gccctgacta acgttactgt tccatacgcg     960
   ctgcaaatcg cgaacaaagg ggcagtaaaa gcgctcgcag acaatacggc actgagagcg    1020
   ggtttaaaca ccgcaaacgg acacgtgacc tatgaagctg tagcaagaga tctaggctat    1080
   gagtatgttc ctgccgagaa agctttacag gatgaatcat ctgtggcggg tgcttaa       1137
<210> 12
   <211> 1380
   <212> DNA
   <213> Chromobacterium violaceum
<400> 12
   atgcagaaac agcgtaccac ctctcagtgg cgtgaactcg atgcggcgca tcatctccat      60
   ccgtttaccg ataccgcgag cctcaatcag gcgggtgcgc gtgtgatgac ccgtggcgaa     120
   ggcgtgtatc tctgggatag cgaaggcaac aaaattattg atggcatggc gggcctctgg     180
   tgcgtgaacg tgggctatgg ccgtaaagat tttgcggaag cggcgcgtcg tcagatggaa     240
   gaactcccgt tttataacac cttctttaaa accacccatc cggcggtggt ggaactcagc     300
   agcctcctcg ccgaagttac cccggcaggt tttgatcgtg tgttttatac caacagcggc     360
   agcgaaagcg tggataccat gattcgtatg gtgcgtcgtt attgggatgt gcagggcaaa     420
   ccggaaaaaa aaaccctcat tggccgttgg aacggctatc acggcagcac cattggcggt     480
   gcgagcctcg gcggcatgaa atatatgcat gaacagggcg atctcccgat tccgggcatg     540
   gcgcatattg aacagccgtg gtggtataaa catggcaaag atatgacccc ggatgaattt     600
   ggcgtggttg cggcgcgttg gctcgaagaa aaaattctcg aaatcggcgc ggataaagtg     660
   gcggcgtttg tgggcgaacc gattcagggt gcgggcggtg tgattgttcc gccggcaacc     720
   tattggccgg aaattgaacg tatttgccgc aaatatgatg tgctcctcgt tgcggatgaa     780
   gtgatttgcg gctttggccg taccggcgaa tggtttggcc atcagcattt tggctttcag     840
   ccggacctct ttaccgcggc gaaaggcctc agcagcggct atctcccgat tggcgcggtg     900
   tttgtgggca aacgtgttgc ggaaggtctc attgcgggcg gtgattttaa ccatggcttt     960
   acctatagcg gccatccggt gtgtgcggcg gtggcgcatg cgaatgttgc ggcgctccgt    1020
   gatgaaggca ttgtgcagcg tgtgaaagat gatattggcc cgtatatgca gaaacgttgg    1080
   cgtgaaacct ttagccgttt tgaacatgtg gatgatgtgc gtggcgtggg catggtgcag    1140
   gcgtttaccc tcgtgaaaaa caaagcgaaa cgtgaactct ttccggattt tggcgaaatt    1200
   ggcaccctct gccgcgatat tttttttcgc aacaacctca ttatgcgtgc gtgcggcgat    1260
   cacattgtgt ctgcaccgcc gctcgttatg acccgtgcgg aagtggatga aatgctcgcc    1320
   gtggcggaac gttgcctcga agaatttgaa cagaccctca aagcgcgtgg cctcgcctaa    1380
<210> 13
   <211> 24
   <212> DNA
   <213> Artificial Sequence
<220>
   <223> Primer
<400> 13
   atgatcatag gggttcctaa agag 24
<210> 14
   <211> 20
   <212> DNA
   <213> Artificial Sequence
<220>
   <223> Primer
<400> 14
   ttaagcaccc gccacagatg 20
<210> 15
   <211> 6866
   <212> DNA
   <213> Artificial Sequence
<220>
   <223> Vector
<400> 15
   atgatcatag gggttcctaa agagataaaa aacaatgaaa accgtgtcgc attaacaccc      60
   gggggcgttt ctcagctcat ttcaaacggc caccgggtgc tggttgaaac aggcgcgggc     120
   cttggaagcg gatttgaaaa tgaagcctat gagtcagcag gagcggaaat cattgctgat     180
   ccgaagcagg tctgggacgc cgaaatggtc atgaaagtaa aagaaccgct gccggaagaa     240
   tatgtttatt ttcgcaaagg acttgtgctg tttacgtacc ttcatttagc agctgagcct     300
   gagcttgcac aggccttgaa ggataaagga gtaactgcca tcgcatatga aacggtcagt     360
   gaaggccgga cattgcctct tctgacgcca atgtcagagg ttgcgggcag aatggcagcg     420
   caaatcggcg ctcaattctt agaaaagcct aaaggcggaa aaggcattct gcttgccggg     480
   gtgcctggcg tttcccgcgg aaaagtaaca attatcggag gaggcgttgt cgggacaaac     540
   gcggcgaaaa tggctgtcgg cctcggtgca gatgtgacga tcattgactt aaacgcagac     600
   cgcttgcgcc agcttgatga catcttcggc catcagatta aaacgttaat ttctaatccg     660
   gtcaatattg ctgatgctgt ggcggaagcg gatctcctca tttgcgcggt attaattccg     720
   ggtgctaaag ctccgactct tgtcactgag gaaatggtaa aacaaatgaa acccggttca     780
   gttattgttg atgtagcgat cgaccaaggc ggcatcgtcg aaactgtcga ccatatcaca 840
   acacatgatc agccaacata tgaaaaacac ggggttgtgc attatgctgt agcgaacatg 900
   ccaggcgcag tccctcgtac atcaacaatc gccctgacta acgttactgt tccatacgcg 960
   ctgcaaatcg cgaacaaagg ggcagtaaaa gcgctcgcag acaatacggc actgagagcg 1020
   ggtttaaaca ccgcaaacgg acacgtgacc tatgaagctg tagcaagaga tctaggctat 1080
   gagtatgttc ctgccgagaa agctttacag gatgaatcat ctgtggcggg tgcttaatta 1140
   aggagatata atatgcagaa acagcgtacc acctctcagt ggcgtgaact cgatgcggcg 1200
   catcatctcc atccgtttac cgataccgcg agcctcaatc aggcgggtgc gcgtgtgatg 1260
   acccgtggcg aaggcgtgta tctctgggat agcgaaggca acaaaattat tgatggcatg 1320
   gcgggcctct ggtgcgtgaa cgtgggctat ggccgtaaag attttgcgga agcggcgcgt 1380
   cgtcagatgg aagaactccc gttttataac accttcttta aaaccaccca tccggcggtg 1440
   gtggaactca gcagcctcct cgccgaagtt accccggcag gttttgatcg tgtgttttat 1500
   accaacagcg gcagcgaaag cgtggatacc atgattcgta tggtgcgtcg ttattgggat 1560
   gtgcagggca aaccggaaaa aaaaaccctc attggccgtt ggaacggcta tcacggcagc 1620
   accattggcg gtgcgagcct cggcggcatg aaatatatgc atgaacaggg cgatctcccg 1680
   attccgggca tggcgcatat tgaacagccg tggtggtata aacatggcaa agatatgacc 1740
   ccggatgaat ttggcgtggt tgcggcgcgt tggctcgaag aaaaaattct cgaaatcggc 1800
   gcggataaag tggcggcgtt tgtgggcgaa ccgattcagg gtgcgggcgg tgtgattgtt 1860
   ccgccggcaa cctattggcc ggaaattgaa cgtatttgcc gcaaatatga tgtgctcctc 1920
   gttgcggatg aagtgatttg cggctttggc cgtaccggcg aatggtttgg ccatcagcat 1980
   tttggctttc agccggacct ctttaccgcg gcgaaaggcc tcagcagcgg ctatctcccg 2040
   attggcgcgg tgtttgtggg caaacgtgtt gcggaaggtc tcattgcggg cggtgatttt 2100
   aaccatggct ttacctatag cggccatccg gtgtgtgcgg cggtggcgca tgcgaatgtt 2160
   gcggcgctcc gtgatgaagg cattgtgcag cgtgtgaaag atgatattgg cccgtatatg 2220
   cagaaacgtt ggcgtgaaac ctttagccgt tttgaacatg tggatgatgt gcgtggcgtg 2280
   ggcatggtgc aggcgtttac cctcgtgaaa aacaaagcga aacgtgaact ctttccggat 2340
   tttggcgaaa ttggcaccct ctgccgcgat attttttttc gcaacaacct cattatgcgt 2400
   gcgtgcggcg atcacattgt gtctgcaccg ccgctcgtta tgacccgtgc ggaagtggat 2460
   gaaatgctcg ccgtggcgga acgttgcctc gaagaatttg aacagaccct caaagcgcgt 2520
   ggcctcgcct aataatctag atcaacaact ctcctggcgc accatcgtcg gctacagcct 2580
   cgggaattgc tgcaagtcga cggatcgccg gaattaattc tcatgtttga cagcttatca 2640
   ctgatcagtg aattaatggc gatgacgcat cctcacgata atatccgggt aggcgcaatc 2700
   actttcgtct ctactccgtt acaaagcgag gctgggtatt tcccggcctt tttgggccgg 2760
   ccggatcccc ccacttcaga agttcctata cactagagaa taggaacttc actatagagt 2820
   cgaataaggg cgacaccccc taattagccc gggcgaaagg cccagtcttt cgactgagcc 2880
   tttcgtttta tttgatgcct ggcagttccc tactctcgca tggggagtcc ccacactacc 2940
   atcggcgcta cggcgtttca cttctgagtt cggcatgggg tcaggtggga ccaccgcgct 3000
   actgccgcca ggcaaacaag gggtgttatg agccatattc aggtataaat gggctcgcga 3060
   taatgttcag aattggttaa ttggttgtaa cactgacccc tatttgttta tttttctaaa 3120
   tacattcaaa tatgtatccg ctcatgagac aataaccctg ataaatgctt caataatatt 3180
   gaaaaaggaa gaatatgagc catattcaac gggaaacgtc gaggccgcga ttaaattcca 3240
   acatggatgc tgatttatat gggtataaat gggctcgcga taatgtcggg caatcaggtg 3300
   cgacaatcta tcgcttgtat gggaagcccg atgcgccaga gttgtttctg aaacatggca 3360
   aaggtagcgt tgccaatgat gttacagatg agatggtcag actaaactgg ctgacggaat 3420
   ttatgccact tccgaccatc aagcatttta tccgtactcc tgatgatgca tggttactca 3480
   ccactgcgat ccccggaaaa acagcgttcc aggtattaga agaatatcct gattcaggtg 3540
   aaaatattgt tgatgcgctg gcagtgttcc tgcgccggtt gcactcgatt cctgtttgta 3600
   attgtccttt taacagcgat cgcgtatttc gcctcgctca ggcgcaatca cgaatgaata 3660
   acggtttggt tgatgcgagt gattttgatg acgagcgtaa tggctggcct gttgaacaag 3720
   tctggaaaga aatgcataaa cttttgccat tctcaccgga ttcagtcgtc actcatggtg 3780
   atttctcact tgataacctt atttttgacg aggggaaatt aataggttgt attgatgttg 3840
   gacgagtcgg aatcgcagac cgataccagg atcttgccat cctatggaac tgcctcggtg 3900
   agttttctcc ttcattacag aaacggcttt ttcaaaaata tggtattgat aatcctgata 3960
   tgaataaatt gcagtttcat ttgatgctcg atgagttttt ctaaaagcgg cgcgccatcg 4020
   aatggcgcaa aacctttcgc ggtatggcat gatagcgccc ggaagagagt caattcaggg 4080
   tggtgaatat gaaaccagta acgttatacg atgtcgcaga gtatgccggt gtctcttatc 4140
   agaccgtttc ccgcgtggtg aaccaggcca gccacgtttc tgcgaaaacg cgggaaaaag 4200
   tggaagcggc gatggcggag ctgaattaca ttcccaaccg cgtggcacaa caactggcgg 4260
   gcaaacagtc gttgctgatt ggcgttgcca cctccagtct ggccctgcac gcgccgtcgc 4320
   aaattgtcgc ggcgattaaa tctcgcgccg atcaactggg tgccagcgtg gtggtgtcga 4380
   tggtagaacg aagcggcgtc gaagcctgta aagcggcggt gcacaatctt ctcgcgcaac 4440
   gcgtcagtgg gctgatcatt aactatccgc tggatgacca ggatgccatt gctgtggaag 4500
   ctgcctgcac taatgttccg gcgttatttc ttgatgtctc tgaccagaca cccatcaaca 4560
   gtattatttt ctcccatgag gacggtacgc gactgggcgt ggagcatctg gtcgcattgg 4620
   gtcaccagca aatcgcgctg ttagcgggcc cattaagttc tgtctcggcg cgtctgcgtc 4680
   tggctggctg gcataaatat ctcactcgca atcaaattca gccgatagcg gaacgggaag 4740
   gcgactggag tgccatgtcc ggttttcaac aaaccatgca aatgctgaat gagggcatcg 4800
   ttcccactgc gatgctggtt gccaacgatc agatggcgct gggcgcaatg cgcgccatta 4860
   ccgagtccgg gctgcgcgtt ggtgcggata tctcggtagt gggatacgac gataccgaag 4920
   atagctcatg ttatatcccg ccgttaacca ccatcaaaca ggattttcgc ctgctggggc 4980
   aaaccagcgt ggaccgcttg ctgcaactct ctcagggcca ggcggtgaag ggcaatcagc 5040
   tgttgccagt ctcactggtg aaaagaaaaa ccaccctggc gcccaatacg caaaccgcct 5100
   ctccccgcgc gttggccgat tcattaatgc agctggcacg acaggtttcc cgactggaaa 5160
   gcgggcagtg actcatgacc aaaatccctt aacgtgagtt acgcgcgcgt cgttccactg 5220
   agcgtcagac cccgtagaaa agatcaaagg atcttcttga gatccttttt ttctgcgcgt 5280
   aatctgctgc ttgcaaacaa aaaaaccacc gctaccagcg gtggtttgtt tgccggatca 5340
   agagctacca actctttttc cgaaggtaac tggcttcagc agagcgcaga taccaaatac 5400
   tgttcttcta gtgtagccgt agttagccca ccacttcaag aactctgtag caccgcctac 5460
   atacctcgct ctgctaatcc tgttaccagt ggctgctgcc agtggcgata agtcgtgtct 5520
   taccgggttg gactcaagac gatagttacc ggataaggcg cagcggtcgg gctgaacggg 5580
   gggttcgtgc acacagccca gcttggagcg aacgacctac accgaactga gatacctaca 5640
   gcgtgagcta tgagaaagcg ccacgcttcc cgaagggaga aaggcggaca ggtatccggt 5700
   aagcggcagg gtcggaacag gagagcgcac gagggagctt ccagggggaa acgcctggta 5760
   tctttatagt cctgtcgggt ttcgccacct ctgacttgag cgtcgatttt tgtgatgctc 5820
   gtcagggggg cggagcctat ggaaaaacgc cagcaacgcg gcctttttac ggttcctggc 5880
   cttttgctgg ccttttgctc acatgttctt tcctgcgtta tcccctgatt ctgtggataa 5940
   ccgtattacc gcctttgagt gagctgatac cgctcgccgc agccgaacga ccgagcgcag 6000
   cgagtcagtg agcgaggaag cggaaggcga gagtagggaa ctgccaggca tcaaactaag 6060
   cagaaggccc ctgacggatg gcctttttgc gtttctacaa actctttctg tgttgtaaaa 6120
   cgacggccag tcttaagctc gggccccctg ggcggttctg ataacgagta atcgttaatc 6180
   cgcaaataac gtaaaaaccc gcttcggcgg gtttttttat ggggggagtt tagggaaaga 6240
   gcatttgtca gaatatttaa gggcgcctgt cactttgctt gatatatgag aattatttaa 6300
   ccttataaat gagaaaaaag caacgcactt taaataagat acgttgcttt ttcgattgat 6360
   gaacacctat aattaaacta ttcatctatt atttatgatt ttttgtatat acaatatttc 6420
   tagtttgtta aagagaatta agaaaataaa tctcgaaaat aataaaggga aaatcagttt 6480
   ttgatatcaa aattatacat gtcaacgata atacaaaata taatacaaac tataagatgt 6540
   tatcagtatt tattatgcat ttagaataaa ttttgtgtcg cccttattcg actcactata 6600
   gaagttccta ttctctagta agtataggaa cttcacttca ttttggatcc ggccggcctg 6660
   cagccccgca gggcctgtct cggtcgatca ttcagcccgg ctcatagata tgcgggcagt 6720
   gagcgcaacg caattaatgt aagttagctc actcattagg caccccaggc ttgacacttt 6780
   atgcttccgg ctcgtataat gtgtggaatt gtgagcggat aacaataaca atttcacaca 6840
   ggatctagga accaaggaga gtggca 6866
<210> 16
   <211> 1857
   <212> DNA
   <213> Oryza sativa
<400> 16
   atgggttctg gtctgttcaa gccgcgtgtt cacccggatc tgcgtgacgt tttctctaaa      60
   atgtctttct tcgacaaaat cggtttcctg ttcatccacg cgttcgacaa acgtaacctg     120
   tggcacaaag ttccggttcc gatcggtctg ctgtacctga acacccgtcg taccctgctg     180
   gaaaaataca atctgctggc cgttggtcgt tcttctcacg gtgcgctgtt cgacccaaaa     240
   gaattcctgt accgtaccga agatggtaaa tacaatgacc cgcacaacgc ggaagccggc     300
   tctcaaaaca ccttctttgg tcgcaacatg gagccggttg accagcagga cgaactgatg     360
   tctccggacc cgttcgttgt tgcgaccaaa ctgctggcgc gtcgtgaata caaagacacc     420
   ggcaaacagt tcaacatcct ggcggcagcg tggatccagt tcatggttca cgattggatg     480
   gaccacatgg aggacaccgg tcaaattggt atcaccgcgc cgaaagaagt tgcgaacgaa     540
   tgcccgctga aatctttcaa attccacccg acgaaagaac tgccgaccaa ctctgacggt     600
   atcaaaatcg gtcactacaa catccgtacc gcgtggtggg acggttctgc ggtttacggt     660
   aacaatgaag aacgtgcgga aaaactgcgt acctacgttg acggtaaact ggttatcggt     720
   gatgacggtc tgctgctgca caaagaaaac ggtgttgcgc tgtctggtga catccgcaac     780
   tcttgggcgg gcgtttctat cctgcaggct ctgttcgtta aagaacacaa cgcggtttgc     840
   gacgcgatca aggaagaaca cccgaacctg tctgacgaag aactgtaccg ttacgcgaaa     900
   ctggttacct ctgcggttat cgcgaaagtt cacaccatcg actggaccgt tgaactgctg     960
   aagaccaaaa ccatgcgtgc ggcgatgcgt gcgaactggt acggcctgct gggtaaaaaa    1020
   atcaaagaca cctttggcca catcggtggt ccgatcctgg gtggtctggt tggtctgaaa    1080
   aaaccaaaca accacggtgt tccgtactct ctgactgaag aattcacctc tgtgtatcgt    1140
   atgcactctc tgatcccgtc taccctgaaa ctgcgtgacc cgaccggtca gccggacgcg    1200
   aataactctc cgccgtgcct ggaagacatc gacatcggtg agatgatcgg tctgaaaggt    1260
   gaggaacagc tgtctaaaat tggcttcgaa aaacaggcgc tgtctatggg ttaccaggcg    1320
   tgtggtgcgc tggaactgtg gaactacccg tctttcttcc gtaacctgat cccacagaac    1380
   ctggacggta ccaatcgttc tgaccgtatc gacctggcgg cgctggaagt ttatcgtgac    1440
   cgtgaacgtt ctgttccgcg ttacaacgaa ttccgtcgtc gtctgttcct gatcccgatc    1500
   aaatcttggg aagacctgac ctctgacaaa gacgcgattg aaaccatccg tgcgatctac    1560
   ggtgacgacg ttgaaaaact ggacctgctg gttggtctga tggcggaaaa gaaaatcaaa    1620
   ggcttcgcga tctctgaaac cgcgttcaac atcttcatcc tgatggcttc tcgtcgtctg    1680
   gaagcggacc gtttcttcac ctccaacttc aacgaagaga cgtacaccaa aaaaggtatg    1740
   cagtgggtta aaaccaccga aggtctgcgc gacgttatca accgtcacta tccggaaatc    1800
   accgcgaaat ggatgaaatc ttcttctgcg ttctctgttt gggacgcgga ctactag        1857
<210> 17
   <211> 5704
   <212> DNA
   <213> Artificial Sequence
<220>
   <223> vector
<400> 17
   ctaggctgct gccaccgctg agcaataact agcataaccc cttggggcct ctaaacgggt      60
   cttgaggggt tttttgctga aacctcaggc atttgagaag cacacggtca cactgcttcc     120
   ggtagtcaat aaaccggtaa accagcaata gacataagcg gctatttaac gaccctgccc     180
   tgaaccgacg accgggtcga atttgctttc gaatttctgc cattcatccg cttattatca     240
   cttattcagg cgtagcacca ggcgtttaag ggcaccaata actgccttaa aaaaattacg     300
   ccccgccctg ccactcatcg cagtactgtt gtaattcatt aagcattctg ccgacatgga     360
   agccatcaca gacggcatga tgaacctgaa tcgccagcgg catcagcacc ttgtcgcctt     420
   gcgtataata tttgcccata gtgaaaacgg gggcgaagaa gttgtccata ttggccacgt     480
   ttaaatcaaa actggtgaaa ctcacccagg gattggctga gacgaaaaac atattctcaa     540
   taaacccttt agggaaatag gccaggtttt caccgtaaca cgccacatct tgcgaatata     600
   tgtgtagaaa ctgccggaaa tcgtcgtggt attcactcca gagcgatgaa aacgtttcag     660
   tttgctcatg gaaaacggtg taacaagggt gaacactatc ccatatcacc agctcaccgt     720
   ctttcattgc catacggaac tccggatgag cattcatcag gcgggcaaga atgtgaataa     780
   aggccggata aaacttgtgc ttatttttct ttacggtctt taaaaaggcc gtaatatcca     840
   gctgaacggt ctggttatag gtacattgag caactgactg aaatgcctca aaatgttctt     900
   tacgatgcca ttgggatata tcaacggtgg tatatccagt gatttttttc tccattttag     960
   cttccttagc tcctgaaaat ctcgataact caaaaaatac gcccggtagt gatcttattt 1020
   cattatggtg aaagttggaa cctcttacgt gccgatcaac gtctcatttt cgccaaaagt 1080
   tggcccaggg cttcccggta tcaacaggga caccaggatt tatttattct gcgaagtgat 1140
   cttccgtcac aggtatttat tcggcgcaaa gtgcgtcggg tgatgctgcc aacttactga 1200
   tttagtgtat gatggtgttt ttgaggtgct ccagtggctt ctgtttctat cagctgtccc 1260
   tcctgttcag ctactgacgg ggtggtgcgt aacggcaaaa gcaccgccgg acatcagcgc 1320
   tagcggagtg tatactggct tactatgttg gcactgatga gggtgtcagt gaagtgcttc 1380
   atgtggcagg agaaaaaagg ctgcaccggt gcgtcagcag aatatgtgat acaggatata 1440
   ttccgcttcc tcgctcactg actcgctacg ctcggtcgtt cgactgcggc gagcggaaat 1500
   ggcttacgaa cggggcggag atttcctgga agatgccagg aagatactta acagggaagt 1560
   gagagggccg cggcaaagcc gtttttccat aggctccgcc cccctgacaa gcatcacgaa 1620
   atctgacgct caaatcagtg gtggcgaaac ccgacaggac tataaagata ccaggcgttt 1680
   cccctggcgg ctccctcgtg cgctctcctg ttcctgcctt tcggtttacc ggtgtcattc 1740
   cgctgttatg gccgcgtttg tctcattcca cgcctgacac tcagttccgg gtaggcagtt 1800
   cgctccaagc tggactgtat gcacgaaccc cccgttcagt ccgaccgctg cgccttatcc 1860
   ggtaactatc gtcttgagtc caacccggaa agacatgcaa aagcaccact ggcagcagcc 1920
   actggtaatt gatttagagg agttagtctt gaagtcatgc gccggttaag gctaaactga 1980
   aaggacaagt tttggtgact gcgctcctcc aagccagtta cctcggttca aagagttggt 2040
   agctcagaga accttcgaaa aaccgccctg caaggcggtt ttttcgtttt cagagcaaga 2100
   gattacgcgc agaccaaaac gatctcaaga agatcatctt attaatcaga taaaatattt 2160
   ctagatttca gtgcaattta tctcttcaaa tgtagcacct gaagtcagcc ccatacgata 2220
   taagttgtaa ttctcatgtt agtcatgccc cgcgcccacc ggaaggagct gactgggttg 2280
   aaggctctca agggcatcgg tcgagatccc ggtgcctaat gagtgagcta acttacatta 2340
   attgcgttgc gctcactgcc cgctttccag tcgggaaacc tgtcgtgcca gctgcattaa 2400
   tgaatcggcc aacgcgcggg gagaggcggt ttgcgtattg ggcgccaggg tggtttttct 2460
   tttcaccagt gagacgggca acagctgatt gcccttcacc gcctggccct gagagagttg 2520
   cagcaagcgg tccacgctgg tttgccccag caggcgaaaa tcctgtttga tggtggttaa 2580
   cggcgggata taacatgagc tgtcttcggt atcgtcgtat cccactaccg agatgtccgc 2640
   accaacgcgc agcccggact cggtaatggc gcgcattgcg cccagcgcca tctgatcgtt 2700
   ggcaaccagc atcgcagtgg gaacgatgcc ctcattcagc atttgcatgg tttgttgaaa 2760
   accggacatg gcactccagt cgccttcccg ttccgctatc ggctgaattt gattgcgagt 2820
   gagatattta tgccagccag ccagacgcag acgcgccgag acagaactta atgggcccgc 2880
   taacagcgcg atttgctggt gacccaatgc gaccagatgc tccacgccca gtcgcgtacc 2940
   gtcttcatgg gagaaaataa tactgttgat gggtgtctgg tcagagacat caagaaataa 3000
   cgccggaaca ttagtgcagg cagcttccac agcaatggca tcctggtcat ccagcggata 3060
   gttaatgatc agcccactga cgcgttgcgc gagaagattg tgcaccgccg ctttacaggc 3120
   ttcgacgccg cttcgttcta ccatcgacac caccacgctg gcacccagtt gatcggcgcg 3180
   agatttaatc gccgcgacaa tttgcgacgg cgcgtgcagg gccagactgg aggtggcaac 3240
   gccaatcagc aacgactgtt tgcccgccag ttgttgtgcc acgcggttgg gaatgtaatt 3300
   cagctccgcc atcgccgctt ccactttttc ccgcgttttc gcagaaacgt ggctggcctg 3360
   gttcaccacg cgggaaacgg tctgataaga gacaccggca tactctgcga catcgtataa 3420
   cgttactggt ttcacattca ccaccctgaa ttgactctct tccgggcgct atcatgccat 3480
   accgcgaaag gttttgcgcc attcgatggt gtccgggatc tcgacgctct cccttatgcg 3540
   actcctgctg gctatggtgg gatttccctt gctgaaatgg gagatccgat catgttcgag 3600
   ctcttattca aatacactgc tgtgttggcg gtaagcgttc tcgagcgcgg ccgcgcgatc 3660
   gcacctggtg tttaaacggc cggcccctgc aggggcagtg agcgcaacgc aattaatgta 3720
   agttagctca ctcattaggc accccaggct tgacacttta tgcttccggc tcgtataatg 3780
   tgtggaattg tgagcggata acaataacaa tttcacacag gatctaggaa ccaaggagag 3840
   tggcatatgg gttctggtct gttcaagccg cgtgttcacc cggatctgcg tgacgttttc 3900
   tctaaaatgt ctttcttcga caaaatcggt ttcctgttca tccacgcgtt cgacaaacgt 3960
   aacctgtggc acaaagttcc ggttccgatc ggtctgctgt acctgaacac ccgtcgtacc 4020
   ctgctggaaa aatacaatct gctggccgtt ggtcgttctt ctcacggtgc gctgttcgac 4080
   ccaaaagaat tcctgtaccg taccgaagat ggtaaataca atgacccgca caacgcggaa 4140
   gccggctctc aaaacacctt ctttggtcgc aacatggagc cggttgacca gcaggacgaa 4200
   ctgatgtctc cggacccgtt cgttgttgcg accaaactgc tggcgcgtcg tgaatacaaa 4260
   gacaccggca aacagttcaa catcctggcg gcagcgtgga tccagttcat ggttcacgat 4320
   tggatggacc acatggagga caccggtcaa attggtatca ccgcgccgaa agaagttgcg 4380
   aacgaatgcc cgctgaaatc tttcaaattc cacccgacga aagaactgcc gaccaactct 4440
   gacggtatca aaatcggtca ctacaacatc cgtaccgcgt ggtgggacgg ttctgcggtt 4500
   tacggtaaca atgaagaacg tgcggaaaaa ctgcgtacct acgttgacgg taaactggtt 4560
   atcggtgatg acggtctgct gctgcacaaa gaaaacggtg ttgcgctgtc tggtgacatc 4620
   cgcaactctt gggcgggcgt ttctatcctg caggctctgt tcgttaaaga acacaacgcg 4680
   gtttgcgacg cgatcaagga agaacacccg aacctgtctg acgaagaact gtaccgttac 4740
   gcgaaactgg ttacctctgc ggttatcgcg aaagttcaca ccatcgactg gaccgttgaa      4800
   ctgctgaaga ccaaaaccat gcgtgcggcg atgcgtgcga actggtacgg cctgctgggt      4860
   aaaaaaatca aagacacctt tggccacatc ggtggtccga tcctgggtgg tctggttggt      4920
   ctgaaaaaac caaacaacca cggtgttccg tactctctga ctgaagaatt cacctctgtg      4980
   tatcgtatgc actctctgat cccgtctacc ctgaaactgc gtgacccgac cggtcagccg      5040
   gacgcgaata actctccgcc gtgcctggaa gacatcgaca tcggtgagat gatcggtctg      5100
   aaaggtgagg aacagctgtc taaaattggc ttcgaaaaac aggcgctgtc tatgggttac      5160
   caggcgtgtg gtgcgctgga actgtggaac tacccgtctt tcttccgtaa cctgatccca      5220
   cagaacctgg acggtaccaa tcgttctgac cgtatcgacc tggcggcgct ggaagtttat      5280
   cgtgaccgtg aacgttctgt tccgcgttac aacgaattcc gtcgtcgtct gttcctgatc      5340
   ccgatcaaat cttgggaaga cctgacctct gacaaagacg cgattgaaac catccgtgcg      5400
   atctacggtg acgacgttga aaaactggac ctgctggttg gtctgatggc ggaaaagaaa      5460
   atcaaaggct tcgcgatctc tgaaaccgcg ttcaacatct tcatcctgat ggcttctcgt      5520
   cgtctggaag cggaccgttt cttcacctcc aacttcaacg aagagacgta caccaaaaaa      5580
   ggtatgcagt gggttaaaac caccgaaggt ctgcgcgacg ttatcaaccg tcactatccg      5640
   gaaatcaccg cgaaatggat gaaatcttct tctgcgttct ctgtttggga cgcggactac      5700
   tagc                                                                   5704